(19)

Europäisches
Patentamt

European
Patent Office

Office européen
des brevets

(11) **EP 3 103 482 A1**

(12) **EUROPEAN PATENT APPLICATION**

(43) Date of publication:
**14.12.2016 Bulletin 2016/50**

(21) Application number: **15305894.6**

(22) Date of filing: **11.06.2015**

(51) Int Cl.:
*A61L 15/32* *(2006.01)*          *A61L 15/34* *(2006.01)*
*C12N 5/00* *(2006.01)*          *A61L 15/52* *(2006.01)*
*A61L 15/42* *(2006.01)*

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicants:
• **Commissariat à l'Énergie Atomique
et aux Énergies Alternatives
75015 Paris (FR)**
• **EURORESEARCH S.r.L.
20122 Milano (IT)**

(72) Inventors:
• **TEXIER-NOGUES, Isabelle
38000 GRENOBLE (FR)**
• **LAGHEZZA-MASCI, Valentina
20122 MILAN (IT)**
• **LAURENZA, Massimo
20122 MILAN (IT)**
• **COURANT, Thomas
51100 REIMS (FR)**
• **SCALESCIANI, Juan Francisco
20122 MILAN (IT)**
• **NAVARRO Y GARCIA, Fabrice
38600 FONTAINE (FR)**

(74) Representative: **Lavoix
2, place d'Estienne d'Orves
75441 Paris Cedex 09 (FR)**

---

(54) **MATERIAL COMPRISING COLLAGEN THE FIBERS OF WHICH ARE COATED WITH NANOPARTICLES**

(57)    The present invention concerns a process for the preparation of a material comprising collagen the fibers of which are coated with nanoparticles, comprising the steps consisting in:

1) providing an aqueous dispersion comprising collagen and nanoparticles comprising an amphiphilic lipid, a solubilising lipid, and a co-surfactant,

2) drying the aqueous dispersion of step 1) to obtain the material comprising collagen the fibers of which are coated with nanoparticles,

the material obtainable by this process and its uses for wound healing dressings, 3D cell culture materials, tissue engineering, as a sensor, in diagnostic, pharmaceutical, or cosmetic industry.

EP 3 103 482 A1

**Description**

The present invention concerns a material derived from collagen, the process for the preparation thereof and the uses thereof, in particular for tissue engineering, 3D cell culture and dressing-design applications.

Collagens are stable, harmless and well tolerated topically and are widely used on an industrial scale as components of formulations, medical devices and cosmetics intended for topical dermatological application. The synthesis of collagen is amplified during the proliferative phase of dermal tissue repair stimulating the proliferation of granulation tissue and accelerating the repair tissue injury. Collagens are described in the scientific literature, the major chemical inventories or in pharmacopoeias (eg European Pharmacopoeia 4th ed. 2002, Martindale, The Extra Pharmacopoeia 31 st ed. 1996).

Collagens are synthesized by fibroblasts and are the major proteins entering the composition of the Extra-Cellular Matrix (ECM), the major structural proteins of any organ, and the most abundant proteins in the body. Collagen exhibits high biocompatibility, biodegradability, and weak antigenicity, which makes it a biomaterial of choice for nanomedicine applications (tissue engineering, 3D cell culture materials, dressings...).

The advantages of collagen in tissue engineering, 3D cell culture and dressing-design applications lay in its fiber-organized structure and the high tensile strength of the fibers. Moreover, it is a material convenient for processing, since it can be prepared and stored in different forms, such as elastic solid, films, gels, sprays, powder... When hydrated or in contact with biological fluids, collagen is a hydrogel material. For storage or easier manipulation, the gel can be dehydrated by lyophilization process. Moreover, the strength of the fibers and strain-resistance of the material can be tuned according to the choice of collagen, and improved by chemical cross-linking.

A few examples of collagen-based dressings are commercially available, for example:

- Promogran@ (Johnson & Johnson, 2001), which is a biocompatible and hemostatic dressing made of lyophilised collagen and regenerated oxidized cellulose,
- Suprasorb® C (Lohmann& Rauscher, 2004), which is an absorbable collagen sponge made of pure open-pore collagen, and
- Biopad® and Condress®, which are equine collagen primary wound dressings for topical use to control minor bleedings and for the management of any kind of ulcer and skin lesion to help wound closure.

Disadvantages of collagen include high cost and variability of the material (extracts of animal origin), as well as its important hydrophilicity which induces rapid release of the material content, as well as fast enzymatic degradation especially in the presence of metalloproteases (MMPs). In this regard, MMPs are over-expressed in an important number of pathologies, especially in wound beads.

The development of an alternative material being less hydrophilic and/or presenting a higher resistance to degradation, while maintaining the advantageous properties of collagen (namely a structure adequate to cell culture and proliferation, its mechanical properties, the easy processing) is therefore needed.

Besides, patent application WO 2010/018223 describes the use of a formulation in the form of a nanoemulsion comprising an aqueous phase and at least one dispersed phase comprising an amphiphilic lipid, a solubilising lipid, a therapeutic agent and a co-surfactant comprising at least one chain composed of alkylene oxide repeat units to deliver an amphiphilic or lipophilic therapeutic agent.

This formulation can be in a gel form by using a specific amount of dispersed phase, as described in application WO 2011/101602, or by covalently linking the nanoparticles of the emulsion, as described in application WO 2013/144369. However, the porosity of these gels is expected to be of the same order of magnitude as the nanoparticles size, i.e. in the nanometric size. This is not well suited for cell colonization, like required for 3D cell culture, tissue engineering or advanced dressing applications, wherein porosity in the micrometric size is required. The development of alternative materials to these gels is therefore required for these applications.

For this purpose, the present invention provides a material comprising collagen the fibers of which are coated with nanoparticles. This material is obtainable by the process described hereafter.

**[Process for the preparation of the material]**

A first object of the present invention is a process for the preparation of a material comprising collagen the fibers of which are coated with nanoparticles, comprising the steps consisting in:

1) providing an aqueous dispersion comprising collagen and nanoparticles comprising:

- an amphiphilic lipid,
- a solubilising lipid comprising at least one fatty acid glyceride, and

- a co-surfactant comprising at least one poly(ethylene oxide) chain,

2) drying the aqueous dispersion of step 1) to obtain the material comprising collagen the fibers of which are coated with nanoparticles.

• Step 1): Providing an aqueous dispersion comprising collagen and nanoparticles

Step 1) of the process consists in providing an aqueous dispersion comprising collagen and nanoparticles. This aqueous dispersion can be obtained by two alternative methods.

According to a first alternative (called method A hereafter), the aqueous dispersion provided in step 1) is prepared by a process comprising the steps consisting in:

A1) providing a nanoemulsion comprising a continuous aqueous phase and nanoparticles comprising the amphiphilic lipid, the solubilising lipid and the co-surfactant,
A2) mixing the nanoemulsion obtained in step A1) with collagen to obtain the aqueous dispersion comprising collagen and nanoparticles.

Thus, according to method A, a nanoemulsion comprising a continuous aqueous phase and nanoparticles (which is the dispersed phase of the nanoemulsion) is first provided and then mixed with collagen. The nanoparticles form the dispersed phase of the nanoemulsion. The nanoemulsion of step A1) is generally free from collagen.
The nanoemulsion of step A1) is typically obtained by a process comprising the following steps:

A1a) preparing an oily phase comprising the solubilising lipid and the amphiphilic lipid,
A1b) preparing an aqueous phase comprising the co-surfactant;
A1c) dispersing the oily phase in the aqueous phase under the action of sufficient shear to form a nanoemulsion,
A1d) optionally recovering the nanoemulsion thus formed.

According to a second alternative (called method B hereafter), the aqueous dispersion provided in step 1) is prepared by a process comprising the steps consisting in:

B1) preparing an oily phase comprising the solubilising lipid and the amphiphilic lipid,
B2) preparing an aqueous phase comprising the co-surfactant and collagen;
B3) dispersing the oily phase in the aqueous phase under the action of sufficient shear to obtain the aqueous dispersion comprising collagen and nanoparticles.

Thus, according to method B, collagen is present in the aqueous phase before formation of the nanoparticles. An aqueous dispersion comprising an aqueous phase comprising collagen and nanoparticles is formed.

• Step A1a) (method A) or step B1) (method B): Preparation of the oily phase

The preparation of the oily phase comprises the mixing of the oily components of the nanoparticles, i.e. at least the solubilising lipid and the amphiphilic lipid.
Mixing can optionally be facilitated by placing one of the constituents or the mixture in solution in a suitable organic solvent. The organic solvent is then evaporated to obtain a homogenous oily phase.
Also, it is preferable to conduct the mixing at a temperature at which all the constituents are liquid.

o Amphiphilic lipid

The oily phase (and thus the nanoparticles of the obtained material) comprises at least one amphiphilic lipid.
To form a stable nanoemulsion, it is necessary to include in the oily phase at least one amphiphilic lipid as surfactant. The amphiphilic nature of the surfactant ensures the stabilisation of the nanoparticles in the continuous aqueous phase.
Generally, the oily phase comprises 0.01 to 99 % by weight, preferably 1 to 60 % by weight, in particular 5 to 25 % by weight of amphiphilic lipid. The quantity of amphiphilic lipid advantageously contributes towards controlling the size of the nanoparticles.

The amphiphilic lipids comprise a hydrophilic part and a lipophilic part. They are generally selected from among compounds whose lipophilic part comprises a saturated or unsaturated, linear or branched chain having 8 to 30 carbon atoms. They can be selected from among phospholipids, cholesterols, lysolipids, sphingomyelins, tocopherols, glucolipids, stearylamines, cardiolipins of natural or synthetic origin; molecules composed of a fatty acid coupled to a hydrophilic group via an ether or ester function such as the esters of sorbitan e.g. sorbitan monooleate and monolaurate marketed under the trade name Span® by Sigma; polymerised lipids; lipids conjugated to short chains of polyethylene oxide (PEG) such as the non-ionic surfactants sold under the trade names Tween® by ICI Americas, Inc. and Triton® by Union Carbide Corp.; sugar esters such as mono- and di-laurate, mono- and di-palmitate, sucrose mono- and distearate; the said surfactants can be used alone or in a mixture.

Phospholipids are the preferred amphiphilic lipids.

Lecithin is a particularly preferred amphiphilic lipid.

o Solubilising lipid

The oily phase (and thus the nanoparticles of the obtained material) also comprises a solubilising lipid comprising at least one fatty acid glyceride.

The solubilising lipid is a lipid having sufficient affinity for the amphiphilic lipid to ease its solubilization. It may be an oil or wax. Preferably the solubilising lipid is solid at room temperature (20°C), but liquid at body temperature (37°C).)

If the amphiphilic lipid is a phospholipid, the solubilising lipid may in particular be a derivative of glycerol and in particular of glycerides obtained by esterification of glycerol with fatty acids.

The preferred solubilising lipid, in particular for the phospholipids, is a mixture of glycerides of fatty acids in particular of saturated fatty acids, and particularly saturated fatty acids having 8 to 18 carbon atoms, more preferably 12 to 18 carbon atoms.

Preferably, the solubilising lipid is a mixture of glycerides of saturated fatty acids comprising at least 10 % by weight of C12 fatty acids, at least 5 % by weight of C14 fatty acids, at least 5 % by weight of C16 fatty acids and at least 5 % by weight of C18 fatty acids.

Preferably, the solubilising lipid is a mixture of glycerides of fatty acids comprising 0 % to 20 % by weight of C8 fatty acids, 0 % to 20 % by weight of C10 fatty acids, 10 % to 70 % by weight of C12 fatty acids, 5 % to 30 % by weight of C14 fatty acids, 5 % to 30 % by weight of C16 fatty acids and 5 % to 30 % by weight of C18 fatty acids.

Particularly preferred are mixtures of semi-synthetic glycerides solid at room temperature sold under the trade name Suppocire®NC by Gattefossé and approved for injection in man. N-type Suppocire® are obtained by direct esterification of fatty acids and glycerol. They are semi-synthetic glycerides of saturated C8 to C18 fatty acids of which the quali-quantitative composition is given in the Table below.

Fatty acid composition of Suppocire® NC by Gattefossé

| Chain length | [weight %] |
|---|---|
| C8 | 0.1 to 0.9 |
| C10 | 0.1 to 0.9 |
| C12 | 25 to 50 |
| C14 | 10 to 24.9 |
| C16 | 10 to 24.9 |
| C18 | 10 to 24.9 |

The aforementioned solubilising lipids allow a stable nanoemulsion to be obtained. Without wishing to be bound by any particular theory, it is assumed that the aforementioned solubilising lipids allow obtaining nanoparticles in the nanoemulsion which have an amorphous core. The core thus obtained has higher internal viscosity without exhibiting any crystallinity. Crystallisation is harmful for the stability of the nanoemulsion since it generally leads to aggregation of the nanoparticles and/or to expelling of the encapsulated molecules outside the nanoparticles. These physical properties promote the physical stability of the nanoemulsion.

The amount of solubilising lipid may vary extensively in relation to the type and amount of amphiphilic lipid contained in the nanoparticles. In general, the oily phase comprises 10 to 100 % by weight, preferably 30 to 90 % by weight and more preferably 50 to 80% by weight of solubilising lipid.

Further to the amphiphilic lipid and to the solubilising lipid, the oily phase prepared in step A1a) (method A) or step B1) (method B) can comprise other components, such as an oil.

o Oil

The oily phase (and thus the nanoparticles of the material) may also comprise one or more other oils.

The oils used preferably have a hydrophilic-lipophilic balance (HLB) of less than 8 and more preferably of between 3 and 6. Advantageously the oils are used without chemical or physical modification prior to the forming of the nanoparticles.

The oils are generally selected from among biocompatible oils, and in particular from among oils of natural origin (vegetable or animal) or synthetic. Among these oils particular mention can be made of oils of natural vegetable origin amongst which are included soybean, flax, palm, groundnut, olive, grape seed and sunflower seed oil; synthetic oils which particularly include triglycerides, diglycerides and monoglycerides. These oils may be first press, refined or inter-esterified oils.

The preferred oils are soybean oil and flax oil.

In general and when present, the oil is contained in the oily phase in a proportion ranging from 5 to 80 % by weight, preferably between 10 and 50 % by weight and more preferably 10 to 30 % by weight.

• Step A1b) (method A) or step B2) (method B): Preparing an aqueous phase

The aqueous phase is preferably water and/or a buffer such as a phosphate buffer e.g. PBS ("Phosphate Buffer Saline") or a saline solution in particular sodium chloride.

The proportion of oily phase and aqueous phase is most variable. However, most often, 1 to 50 %, preferably 5 to 40 % and more particularly 10 to 35 % by weight of oily phase compared to the total weight of the oily phase and of the aqueous phase (without taking into account the collagen weight in the aqueous phase weight as regards step B2)).

The aqueous phase comprises a co-surfactant. In step A1b) (method A), the aqueous phase is generally free from collagen. On the contrary, in step B2) (method B), the aqueous phase comprises collagen.

o Co-surfactant

The aqueous phase comprises a co-surfactant which allows stabilization of the nanoparticles.

Although the co-surfactant is introduced in the aqueous phase, the co-surfactant is located in the nanoparticles obtained at the end of steps A1c) or step B3). In the present application, the weight of nanoparticles thus includes the weight of co-surfactant.

The co-surfactant is generally a water-soluble surfactant. The co-surfactant comprises at least one poly(ethylene oxide) chain. Preferably, the number of ethylene oxide units in the chain varies between 2 and 500, such as from 25 to 400.

As examples of co-surfactants particular mention can be made of the conjugated compounds polyethylene glycol/phosphatidyl-ethanolamine (PEG-PE), the ethers of fatty acid and of polyethylene glycol such as the products sold under the trade names Brij® (for example Brij® 35, 58, 78 or 98) by ICI Americas Inc., the esters of fatty acid and of polyethylene glycol such as the products sold under the trade names Myrj® by ICI Americas Inc. (for example Myrj® 45, 52, 53 ou 59) and the block copolymers of ethylene oxide and propylene oxide such as the products sold under the trade names Pluronic® by BASF AG (for example Pluronic® F68, F127, L64, L61, 10R4, 17R2, 17R4, 25R2 or 25R4) or the products sold under the trade name Synperonic® by Unichema Chemie BV (for example Synperonic® PE/F68, PE/L61 or PE/L64). Preferably, the co-surfactant is an ester of fatty acid and of polyethylene glycol.

The aqueous phase generally comprises 0.01 to 50 % by weight, preferably 1 to 30 % by weight, and in particular 5 to 25 % by weight of a co-surfactant.

o Collagen (as regards the aqueous phase of step B2) (method B))

There are different types of collagen, distinct from the chemical and structural point of view, including the native collagen of type I, which represents 70% of the collagen of the dermal matrix and is essential in tissue reconstruction.

Typically, the collagen used in step B2) is undenaturated collagen, i.e. collagen of type I. Preferably, the collagen is extracted from horse tendon.

The collagen used in step B2) is preferably in a gel form. Typically, the collagen gel comprises (or is constituted from) collagen and an aqueous solution, such as a buffer, for example an acetate buffer. Preferably, the collagen gel comprises from 0,1 to 10% by weight of collagen, more preferably from 0.5 to 2.0% by weight, for example 1% by weight. In another embodiment, the collagen used in step B2) is in solid form.

Generally, the weight of collagen used in step B2) is calculated so that the weight of collagen compared to the total weight of the nanoparticles components (wherein the nanoparticles components comprise the amphiphilic lipid, the solubilising lipid, the co-surfactant, the optional oil) is from 0.05/1 to 1/1, preferably from 0.05/1 to 0.2/1. Of course, when the collagen used in step B2) is preferably in a gel form, the weight of collagen above corresponds to dry collagen.

o Thickening agent

The aqueous phase may further comprise a thickening agent such as glycerol, a saccharide, oligosaccharide or polysaccharide, a gum or a protein, preferably glycerol. The use of an aqueous phase of higher viscosity facilitates emulsification and thereby allows a reduction in sonication time.

The aqueous phase advantageously comprises 0 to 50 % by weight, preferably 1 to 30 % by weight and more particularly 5 to 30 % by weight of thickening agent.

o Other additives

The aqueous phase may further contain other additives such as dyes, stabilisers, pH adjusting agent and/or preserving agents. For example, the aqueous phase comprises acetic acid which is useful as preservative and as pH adjuster. Preferably, the pH of the aqueous phase of step A1) or of step B2) is from 2.5 to 5.0, more preferably from 3.0 to 4.5, for example from 3.5 to 4.0.

• Step A1c) (method A) or step B3) (method B): Dispersing the oily phase in the aqueous phase

Step A1c) (method A) or step B3) (method B) consists in dispersing the oily phase in the aqueous phase under the action of sufficient shear to form nanoparticles.

Advantageously the oily phase is dispersed in the aqueous phase in the liquid state. If one of the phases solidifies at room temperature (20°C), it is preferable to perform mixing by heating one or preferably both phases to melting temperature or higher.

Forming the nanoparticles under shear effect is preferably performed using a sonicator, a microfluidiser or a high pressure homogenizer.

Forming the nanoparticles under shear effect in step A1c) (method A) is preferably performed using a sonicator or a high pressure homogenizer. For sonication, preferably the aqueous phase and then the oily phase are added in the desired proportions to a cylindrical container and the sonicator is immersed in the centre thereof and set in operation for sufficient time to obtain nanoparticles, most often for a few minutes. For High Pressure Homogenization, typically, the aqueous phase and the oily phase are crudely mixed in order to pre-form a coarse emulsion, which is then used to fill the tank of the homogeneizer. The emulsion is then processed with the homogeneizer using the number of cycles necessary to achieve the targeted size for the nanoparticles.

Forming the nanoparticles under shear effect in step B3) (method B) is preferably performed using a high pressure homogenizer. Indeed, a degradation of the collagen fibers might be observed when a sonicator is used instead.

At the end of step A1c) (method A) or step B3) (method B), the diameter of the obtained nanoparticles is generally greater than 10 nm and smaller than 500 nm, preferably between 20 and 400 nm, most preferably between 30 and 300 nm.

At the end of step A1c) (method A), it can be advantageous that the obtained nanoemulsion displays an absolute value of the zeta potential of the surface of the nanoparticles lower than 20 mV, preferably when the aqueous phase is PBS 0.1 x.

For example, the mean diameter and/or zeta potential can be determined by dynamic light scattering (DLS), for example with a Zeta Sizer Nano ZS Malvern Instrument. • Step A2) (method A): mixing the nanoemulsion obtained in step A1) with collagen to obtain the aqueous dispersion comprising collagen and nanoparticles.

Typically, the collagen used in step A2) is undenatured collagen, i.e. collagen of type I. Preferably, the collagen is extracted from horse tendon.

The collagen used in step A2) is preferably in a gel form. Typically, the collagen gel comprises (or is constituted from) collagen and an aqueous solution, such as a buffer, for example an acetate buffer. Preferably, the collagen gel comprises from 0,1 to 10% by weight of collagen, more preferably from 0.5 to 2.0% by weight. In another embodiment, the collagen is in solid form.

Generally, the weight of collagen used in step A2) is calculated so that the weight of collagen compared to the total weight of the nanoparticles components (wherein the nanoparticles components comprise the amphiphilic lipid, the solubilising lipid, the co-surfactant, the optional oil) is from 0.5/1 to 1000/1, preferably from 1/1 to 500/1, most preferably from 2/1 to 500/1. Of course, when the collagen used in step A2) is preferably in a gel form, the weight of collagen above corresponds to dry collagen.

• Optional subsequent steps after step A2) (method A)

The aqueous dispersion obtained at the end of step A2) (method A) can be purified, for example by column purification or dialysis.

The dispersion can be diluted and/or sterilised, for example by filtration or dialysis. This step allows the removal of any aggregates which may have been formed during preparation of the nanoparticles.

• Optional step 1bis): Applying the dispersion of step 1) to a substrate

In one embodiment, the process comprises between steps 1) and 2) a step 1bis) of applying the dispersion of step 1) to a substrate. For example, glass substrates or polymeric substrates such as polyurethane foam substrates can be used.

The application of the dispersion can be carried out by spraying, coating, rolling...

The dispersion of step 1) can be applied to the totality or a part of the surface of the substrate. It is thus possible to apply the dispersion to a specific pattern, the form of which being adjustable depending on the intended use of the material.

• Step 2): Drying the aqueous dispersion of step 1) to obtain the material comprising collagen the fibers of which are coated with nanoparticles

Step 2) of the process consists in drying the aqueous dispersion of step 1). Preferably, drying is carried out by freeze drying (also called lyophilisation). This allows obtaining a spongy material.

Generally, the material obtained at the end of step 2) comprises less than 25%wt, preferably less than 20% of water. The water proportion can be determined using a Karl Fisher apparatus.

In the embodiment wherein the process comprises step 1bis), the material obtained at the end of step 2) is supported on the substrate.

**[Material]**

A second object of the invention is the material comprising collagen the fibers of which are coated with nanoparticles obtainable (or obtained) by the process described above.

Generally, the material is a porous material. The material is in the form of a sponge. Typically, the material comprises pores the Feret's diameter of which are between 50 and 150 $\mu$m. The Feret's diameter can be determined using SEM Scanning Electron Microscopy (SEM) images, for example images obtained from a JEOL JSM 6010LA electron microscope and then using a software to analyze the diameters of the pores, for example the ImageJ® software. A fibrous structure with an average pore size of 50-150 $\mu$m is an ideal structure for collagen material for cell growth (C. J. Doillon, C. F. Whyne, S. NBrandwein, F. H. Silver. "Collagen-based wound dressings: control of the pore structure and morphology". J. Biomed. Mater. Res. , vol.20, pp.1219-1228, 1986).

Generally, the material according to the invention comprises less than 25%wt, preferably less than 20%wt of water. The water proportion can be determined using a Karl Fisher apparatus.

Advantageously, the swelling ratio (SR) of the material in 1X PBS is close to the one of the collagen it comprises. Generally, the swelling ratio of the material in 1X PBS is from 70 to 100%, preferably from 80 to 100%, more preferably from 90 to 100% of the swelling ratio of collagen (It being understood that collagen is the same than the collagen comprised in the material. For example if the material comprises Type I collagen, the swelling ratio of the material is from 70 to 100% of the swelling ratio of Type I collagen). The swelling ratio can be calculated according to the following equation:

$$SR = 100 \times (W_w - W_d) / W_d$$

in which $W_d$ is the weight of material (dry material) and $W_w$ is the material after 24 hours immersion in either water or 1X PBS.

Generally, the weight of collagen in the material compared to the weight of the nanoparticles (i.e. the total weight of the nanoparticles components, wherein the nanoparticles components comprise the amphiphilic lipid, the solubilising lipid, the co-surfactant, the optional oil) is from 0.05/1 to 1000/1, preferably from 0.1/1 to 500/1, most preferably from 2/1 to 500/1.

The nanoparticles of the material according to the invention comprise the components used for their preparation and described above, i.e. a solubilising lipid, an amphiphilic lipid, a co-surfactant, and optionally the other components described above (an oil for example).

The mean diameter of the nanoparticles of the material is generally the same than the diameter of the nanoparticles formed in step A1c) or B3), i.e. greater than 10 nm and smaller than 500 nm, preferably between 20 and 400 nm, most preferably between 30 and 300 nm.

In the material according to the invention, the distribution of the nanoparticles within the collagen fiber matrix is homogeneous. As the nanoparticles are smaller than collagen fibers, the nanoparticles coat the collagen fiber matrix without disturbing the arrangement of the fibers. The nanoparticles are coated on the collagen fibers and are regularly distributed. In the material, the nanoparticles present a spherical shape. The collagen structure and its porosity are not modified since the nanoparticles are very small in comparison to collagen fibers. However, the physicochemical properties of the collagen fiber matrix are modified by this coating. Advantageously, the material according to the invention:

- is less hydrophilic than collagen, and/or
- exhibits a higher resistance to degradation than collagen (in particular when in contact with cells), and/or
- presents a more elastic behavior than collagen,

while maintaining the structure and porosity of collagen.

Advantageously, the material is not cytotoxic.

The material can be supported on a substrate.

A third object of the invention is a material comprising collagen the fibers of which are coated with nanoparticles comprising a solubilising lipid comprising at least one fatty acid glyceride, an amphiphilic lipid, and a co-surfactant comprising at least one poly(ethylene oxide) chain, wherein the material comprises pores the Feret's diameter of which are between 50 and 150 $\mu$m. This material can have the features described above for the material obtainable by the process.

**[Use of the material]**

A fourth object of the invention is the use of the material comprising collagen the fibers of which are coated with nanoparticles described above for wound healing dressings, 3D cell culture materials, tissue engineering, as a sensor, in diagnostic, pharmaceutical, or cosmetic industry.

A fifth object of the invention is a wound healing dressing, a 3D cell culture material, or a tissue comprising the material comprising collagen the fibers of which are coated with nanoparticles described above.

As regards the use of the material as a wound healing dressing, the nanoparticles coated on the collagen fibers of the material advantageously possess by themselves beneficial effects for skin and tissue repair.

**[Definitions]**

In the present application, by the term « nanoemulsion » is meant a composition having at least two phases, an oily phase and an aqueous phase in which the mean size of the nanoparticles of the dispersed phase is smaller than 1 micron, preferably 10 to 500 nm and in particular 20 to 400 nm, and most preferably 30 to 300 nm. The nanoemulsion is an oil-in-water emulsion. It can be simple or double (i.e. the dispersed phase may comprise an internal aqueous phase). It is preferably simple.

In the meaning of the present application, the expression « dispersed phase » designates the nanoparticles comprising the solubilising lipid / the amphiphilic lipid / the co-surfactant / the optional oil. The dispersed phase is generally free of aqueous phase.

The term « nanoparticle » encompasses both the nanoparticles (also called droplets) of liquid oil and the solid nanoparticles derived from emulsions of oil-in-water type in which the dispersed phase is solid. In the present application, the term "nanoparticle" is used both for the nanoparticles of the dispersed phase of the nanoemulsion/aqueous dispersion and for the nanoparticles coating the collagen fibers after the drying step (i.e. in the absence of aqueous phase).

The term « lipid » in this description designates all fatty bodies or substances containing fatty acids present in the fats of animal origin and in vegetable oils. They are hydrophobic or amphiphilic molecules chiefly formed of carbon, hydrogen and oxygen and having a density lower than that of water. The lipids may be in the solid state at room temperature (25°C) as in waxes, or liquid state as in oils.

The term « phospholipid » concerns lipids having a phosphate group, in particular phosphoglycerides. Most often the phospholipids comprise a hydrophilic end formed by the optionally substituted phosphate group and two hydrophobic ends formed by fatty acid chains. Amongst the phospholipids, particular mention is made of phosphatidylcholine, phosphatidyl ethanolamine, phophatidyl, inositol, phosphatidyl serine and sphingomyeline.

The term « lecithin » designates phosphatidylcholine i.e. a lipid formed from a choline, a phosphate, a glycerol and from two fatty acids. It more broadly covers living phospholipid extracts of vegetable or animal origin, provided that they are mostly formed of phosphatidylcholine. These lecithins generally form mixtures of lecithins carrying different fatty acids.

The term « fatty acid » designates aliphatic carboxylic acids having a carbon chain of at least 4 carbon atoms.

Natural fatty acids have a carbon chain of 4 to 28 carbon atoms (generally an even number). The term long chain fatty acid is used for a length of 14 to 22 carbons and very long chain if there are more than 22 carbons.

By the term « surfactant » is meant compounds with amphiphilic structure imparting particular affinity thereto for interfaces of oil/water and water/oil type providing them with the capability of reducing the free energy of these interfaces and stabilising dispersed systems.

By the term « co-surfactant » is meant surfactant acting in addition to a surfactant to further reduce interface energy.

The invention is descried in better detail with reference to the appended Figures and following examples.

**FIGURES**

- Figures 1-6: SEM images of cuts of the material obtained in 1.5

    Figures 1 and 2: collagen (free from nanoparticles), longitudinal profile orientation of the material
    Figures 3 and 4: collagen loaded with F50 nanoparticles (collagen/lipid 10/1 weight/weight), longitudinal profile orientation of the material
    Figures 5 and 6: collagen loaded with F120 nanoparticles (collagen/lipid 10/1 weight/weight), longitudinal profile orientation of the material

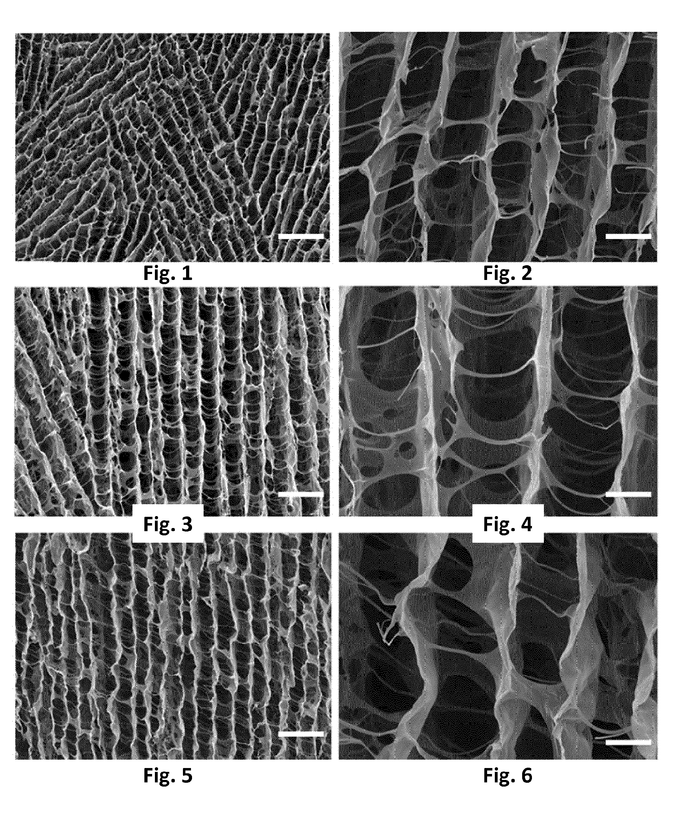

    Figures 1, 3 and 5: the magnification was 180 (the scale bar represents 100 $\mu$m)
    Figures 2, 4, 6: the magnification was 850 (the scale bar represents 20 $\mu$m).

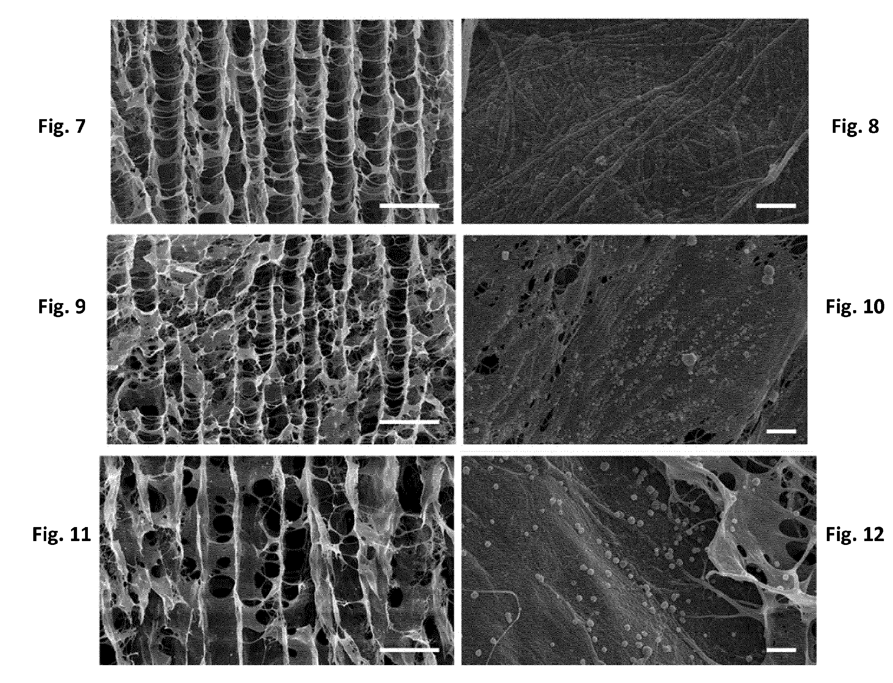

- Figures 7-12: SEM images of cuts of the material obtained in 1.5

    Figures 7 and 8: collagen loaded with F50 nanoparticles with a collagen/lipid ratio 10/1 weight/weight (longitudinal profile orientation of the material)
    Figures 9 and 10: collagen loaded with F50 nanoparticles with a collagen/lipid ratio 3.3/1 weight/weight (longitudinal profile orientation of the material) Figures 11 and 12: collagen loaded with F50 nanoparticles with a collagen/lipid ratio 2/1 weight/weight (longitudinal profile orientation of the material) Figures 7, 9 and 11: the magnification was 250 (the scale bar represents 100 $\mu$m);
    Figure 8: the magnification was 7,000 (the scale bar represents 2 $\mu$m);
    Figures 10 and 12: the magnification was 1,000 (the scale bar represents 10 $\mu$m).

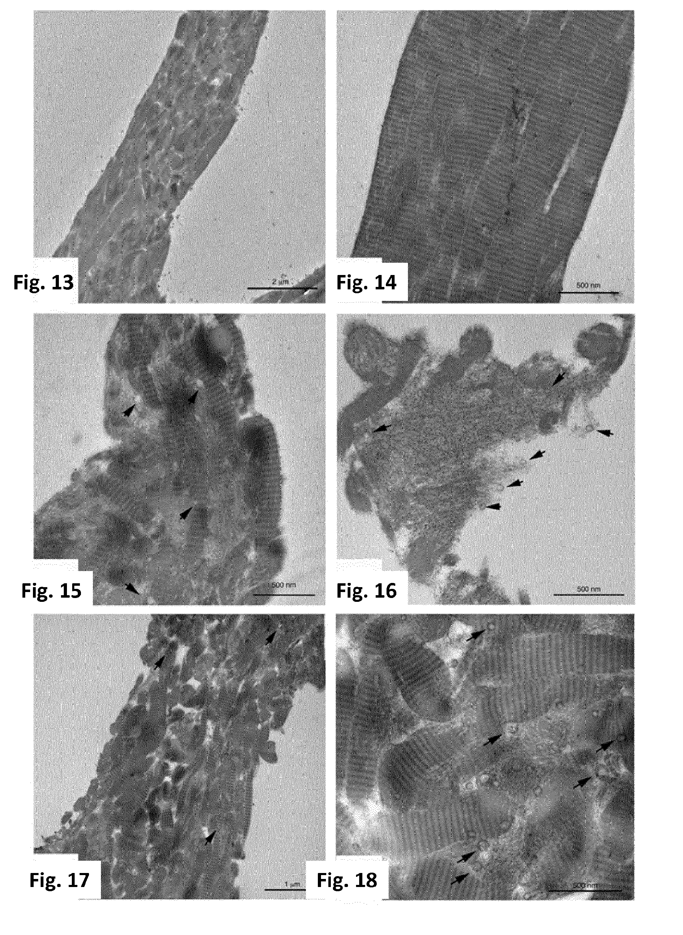

- Figures 13-18:TEM images of the materials obtained in example 1.5. Arrows point at some of well-evidenced nanoparticles.

    Figures 13 and 14: collagen (free from nanoparticles):
    Figure 13: the scale bar represents 2 $\mu$m,
    Figure 14: the scale bar represents 500 nm;
    Figures 15 and 16: collagen loaded with F50 nanoparticles with collagen/lipid 10/1 weight/weight; the scale bar represents 500 nm;
    Figures 17 and 18: collagen loaded with F120 nanoparticles with collagen/lipid 10/1 weight/weight;
    Figure 17: the scale bar represents 1 $\mu$m;
    Figure 18: the scale bar represents 500 nm.

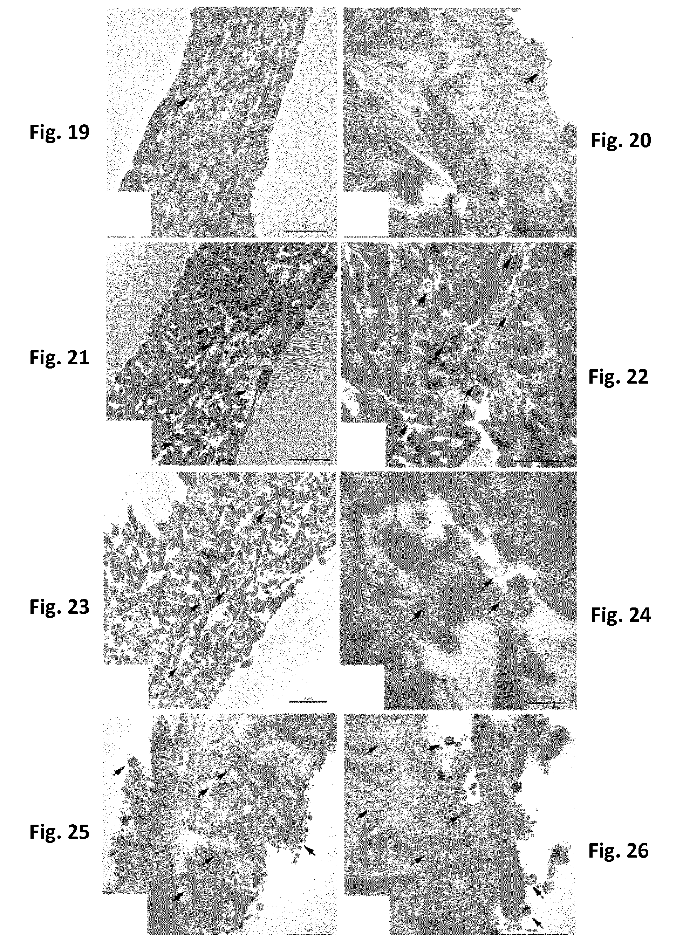

- Figures 19-26:TEM images of the collagen materials loaded with F50 nanoparticles obtained in example 1.5. Arrows point at some of well-evidenced nanoparticles.

    Figures 19 and 20: collagen loaded with F50 nanoparticles with collagen/lipid 5/1 weight/weight;
    Figure 19: the scale bar represents 1 $\mu$m,
    Figure 20: the scale bar represents 500 nm;
    Figures 21 and 22: collagen loaded with F50 nanoparticles with collagen/lipid 3.3/1 weight/weight;
    Figure 21: the scale bar represents 2 $\mu$m;
    Figure 22: the scale bar represents 1 $\mu$m.
    Figures 23 and 24: collagen loaded with F50 nanoparticles with collagen/lipid 2.5/1 weight/weight;
    Figure 23: the scale bar represents 2 $\mu$m,
    Figure 24: the scale bar represents 200 nm;

Figures 25 and 26: collagen loaded with F50 nanoparticles with collagen/lipid 2/1 weight/weight;
Figure 25: the scale bar represents 1 $\mu$m;
Figure 26: the scale bar represents 500 nm.

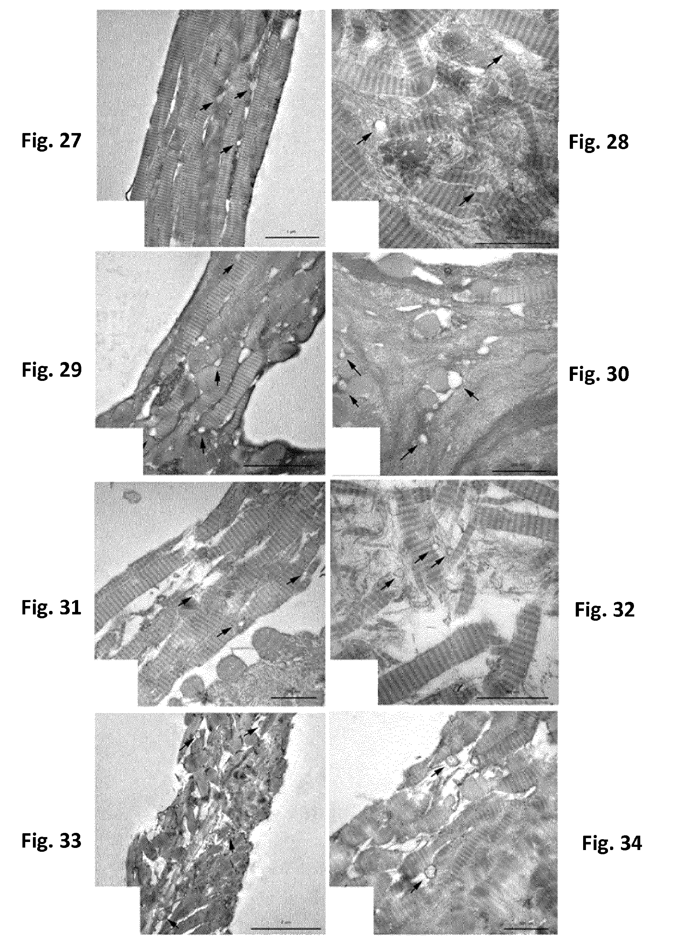

- Figures 27-34:TEM images of the collagen materials loaded with F120 nanoparticles obtained in example 1.5. Arrows point at some of well-evidenced nanoparticles.

Figures 27 and 28: collagen loaded with F120 nanoparticles with collagen/lipid 5/1 weight/weight;
Figure 27: the scale bar represents 1 $\mu$m,
Figure 28: the scale bar represents 500 nm;
Figures 29 and 30: collagen loaded with F120 nanoparticles with collagen/lipid 3.3/1 weight/weight;
Figure 29: the scale bar represents 1 $\mu$m;
Figure 30: the scale bar represents 500 nm.
Figures 31 and 32: collagen loaded with F120 nanoparticles with collagen/lipid 2.5/1 weight/weight;
Figure 31: the scale bar represents 500 $\mu$m,
Figure 32: the scale bar represents 500 nm;
Figure 33-34: collagen loaded with F120 nanoparticles with collagen/lipid 2/1 weight/weight;
Figure 33: the scale bar represents 2 $\mu$m;
Figures 34: the scale bar represents 500 nm.

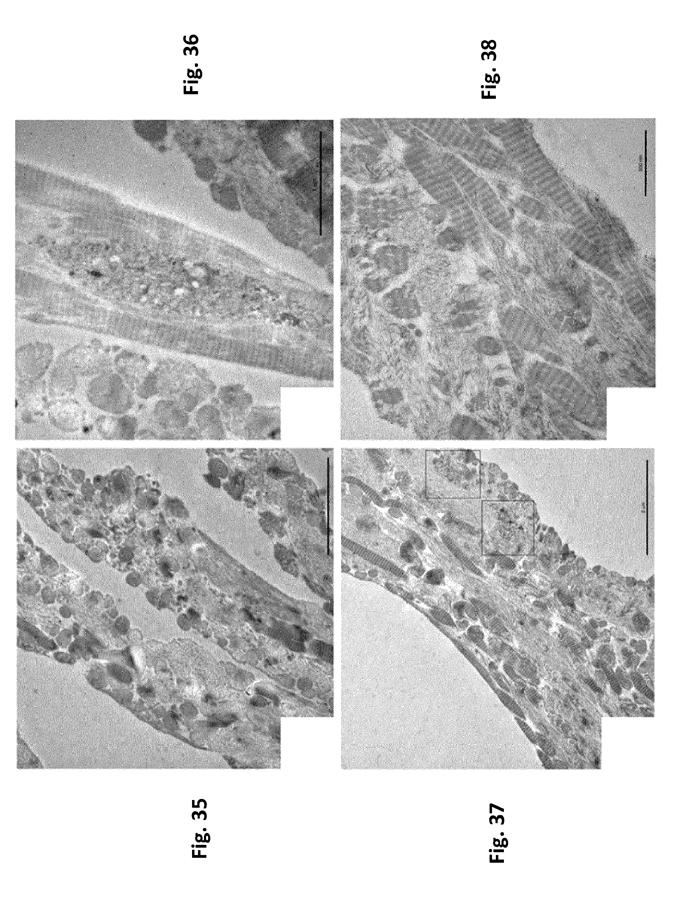

- Figures 35-38:TEM images of the materials obtained in example 1.5.

Figures 35 and 36: collagen/Myrj™ s40 material 21/1 weight/weight:
Figure 35: the scale bar represents 2 $\mu$m,
Figure 36: the scale bar represents 1 $\mu$m;
Figures 37 and 38: collagen/Myrj™ s40 material 40/1 weight/weight:
Figure 37: the scale bar represents 2 $\mu$m,
Figure 38: the scale bar represents 500 nm.

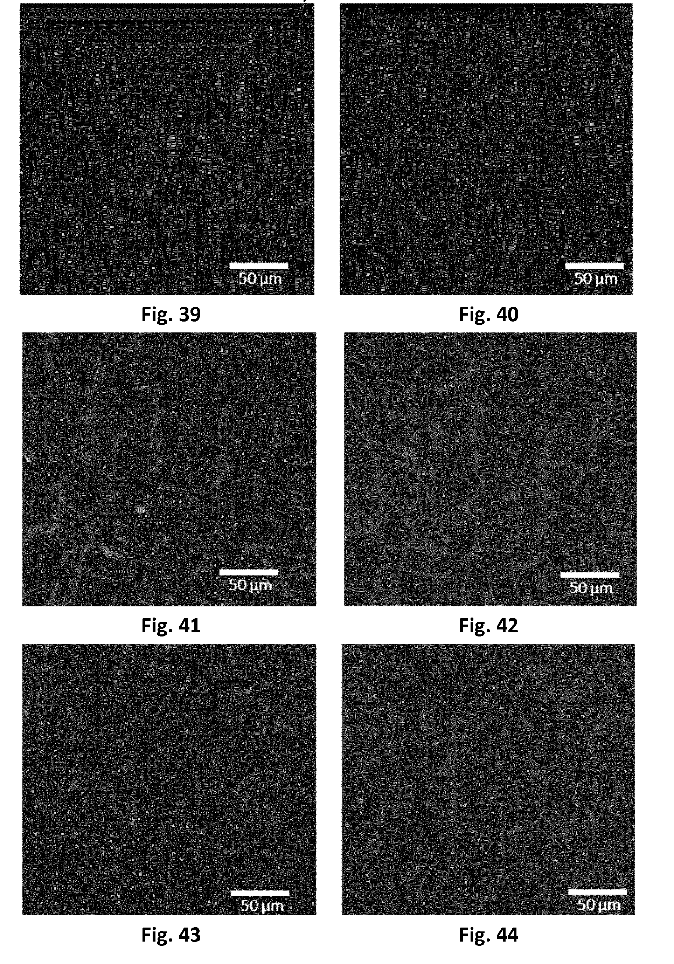

- Figures 39-44: Confocal fluorescence microscopy images of the collagen 100% material (control - figures 39 and 40), collagen/FRET-F50 particles 10/1 material (Figures 41 and 42), and collagen/FRET-F120 particles 10/1 material (Figures 43 and 44). The scale bar represents 50 $\mu$m.

Figures 39, 41 and 43 are images obtained in the DiI channel (excitation in 555-620 nm range);
Figures 40, 42 and 44 are images obtained in the DiD channel (excitation in 650-750 nm range).

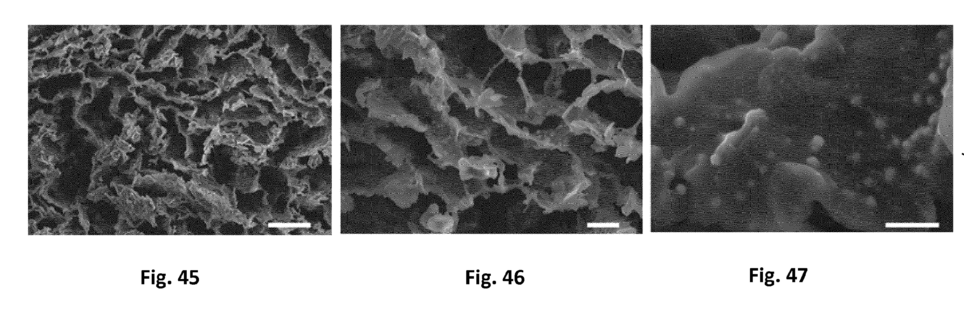

- Figures 45-47: SEM images of cuts of the material obtained in 1.8.

Figure 45: the magnification was 190, the scale bar represents 100 $\mu$m;
Figure 46: the magnification was 600, the scale bar represents 20 $\mu$m;
Figure 47: the magnification was 2,200, the scale bar represents 10 $\mu$m.

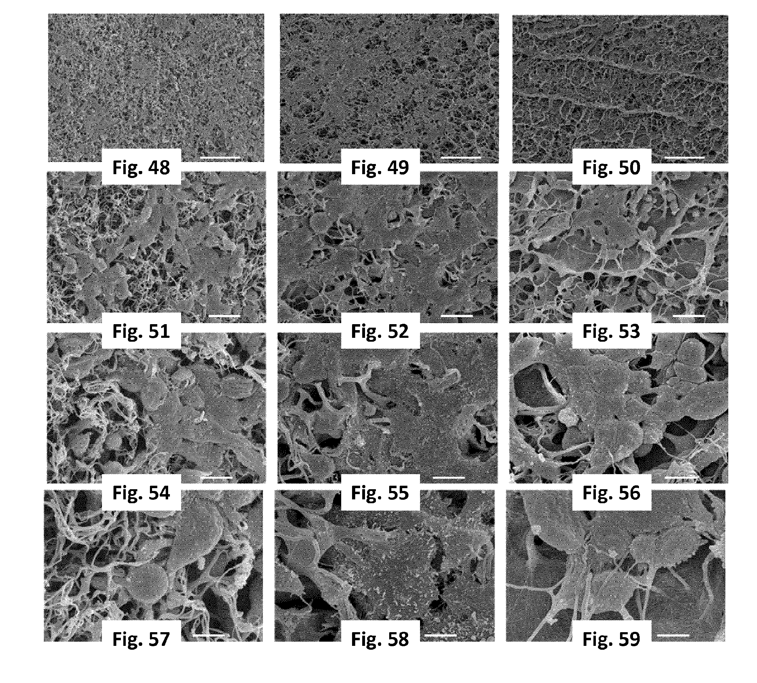

- Figures 48-59: SEM images of HaCat cells 3 days after seeding on collagen (Figures 48, 51, 54 and 57), on collagen/F50 nanoparticle 10/1 material (Figures 49, 52, 55 and 58) or on collagen/F120 nanoparticle 10/1 material (Figures 50, 53, 56 and 59) (prepared according to example 1.5). The images were taken with different magnifications: 220 (Figures 48-50, scale bar represents 100 $\mu$m); 850 (Figures 51-53, scale bar represents 20 $\mu$m); 1,800 (Figures 54-56, scale bar represents 10 $\mu$m); 3,000 (left), 3,700 (center), 3,300 (right) (Figures 57-59, scale bar represents 5 $\mu$m).

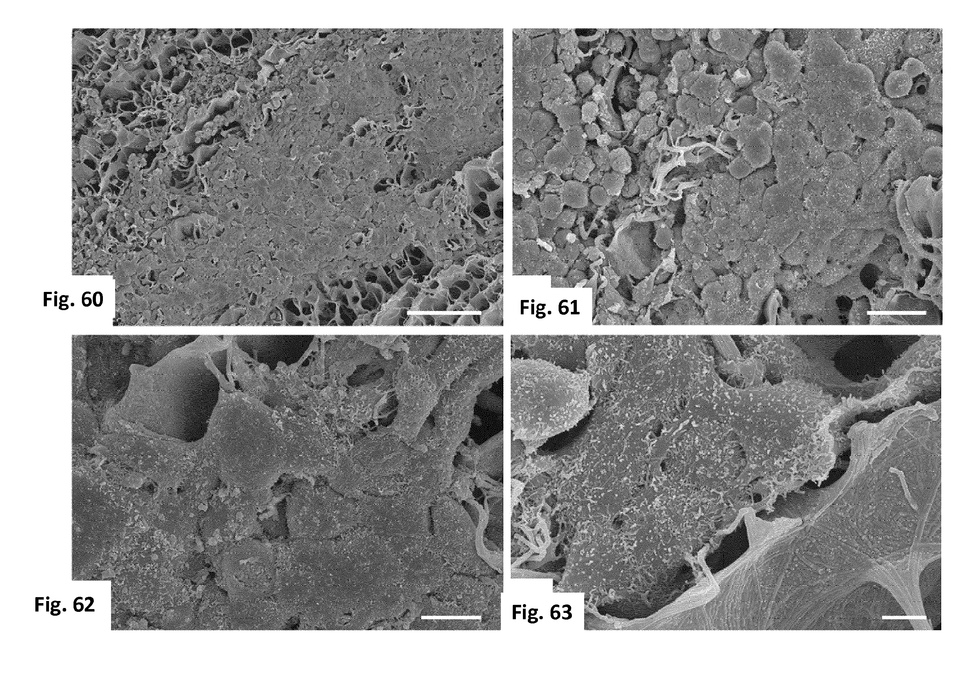

- Figures 60-63: SEM images of HaCat cells 7 days after seeding on collagen/F120 nanoparticle 10/1 material (prepared according to example 1.5). The images were taken with different magnifications: Figure 60: 220 (scale bar represents 100 $\mu$m); Figure 61: 850 (scale bar represents 20 $\mu$m); Figure 62: 1,800 (scale bar represents 10 $\mu$m); Figure 63: 3,000 (scale bar represents 5 $\mu$m).

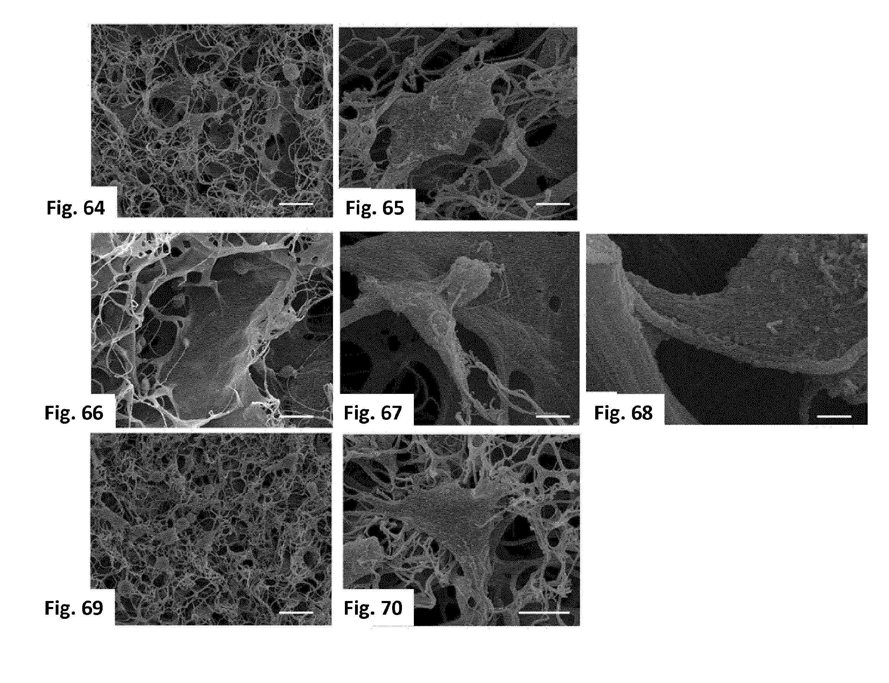

- Figures 64-70: SEM images of NIH 3T3 cells 4 hours after seeding on collagen (Figures 64 and 65), on collagen/F50 nanoparticle 10/1 material (Figures 66 to 68) or on collagen/F120 nanoparticle 10/1 material (Figures 69 and 70) (prepared according to example 1.5). The images were taken with different magnifications:

Figures 64, 66 and 69: 750 (scale bar represents 20 $\mu$m);
Figures 65 and 67: 3,300 (scale bar represents 5 $\mu$m);

Figure 70: 2,300 (scale bar represents 10 $\mu$m);
Figure 68: 14,000 (scale bar represents 1 $\mu$m).

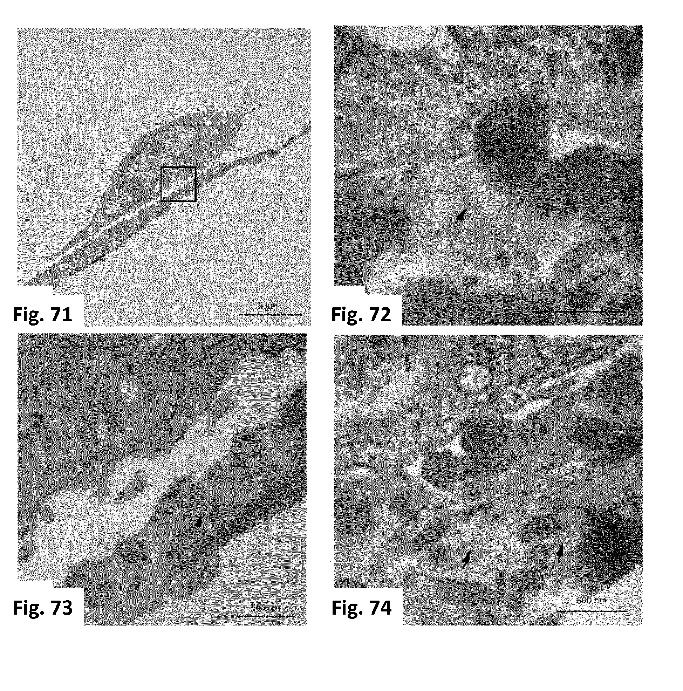

- Figures 71-74: TEM images of NIH 3T3 cells 4 hours after seeding on collagen/F50 nanoparticle 10/1 material. 50 nm particles are evidenced by arrows. Figure 73 is an enlargement of Figure 71 (square).

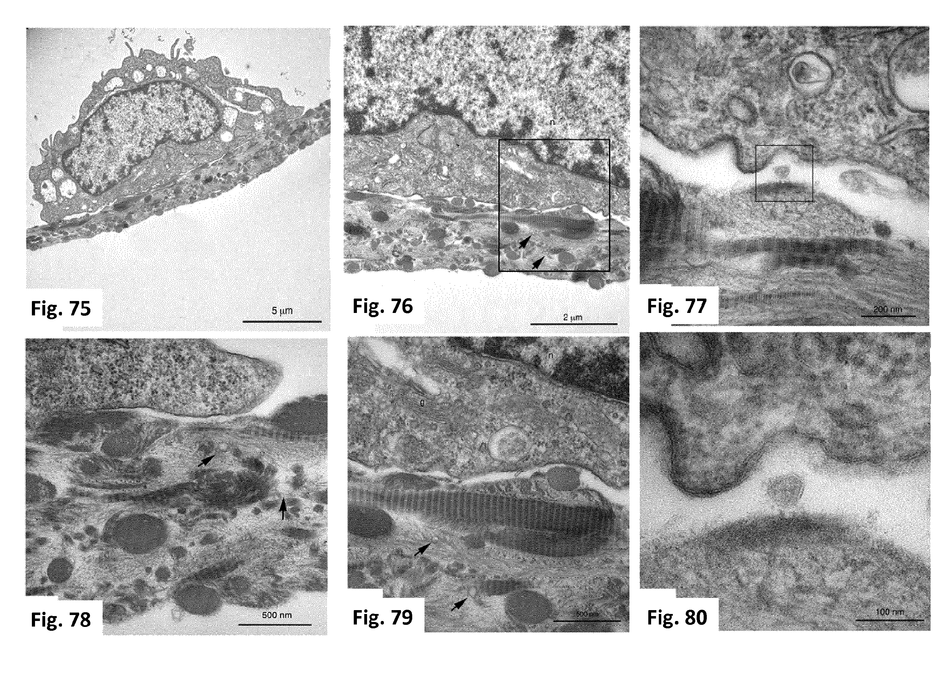

- Figures 75-80: TEM images of NIH 3T3 cells 4 hours after seeding on collagen/F120 nanoparticle 10/1 material. 120 nm particles are evidenced by arrows. n indicates cell nucleus, g cell Golgi. Figure 79 and Figure 80 are enlargements of Figure 76 and Figure 77 respectively (squares).

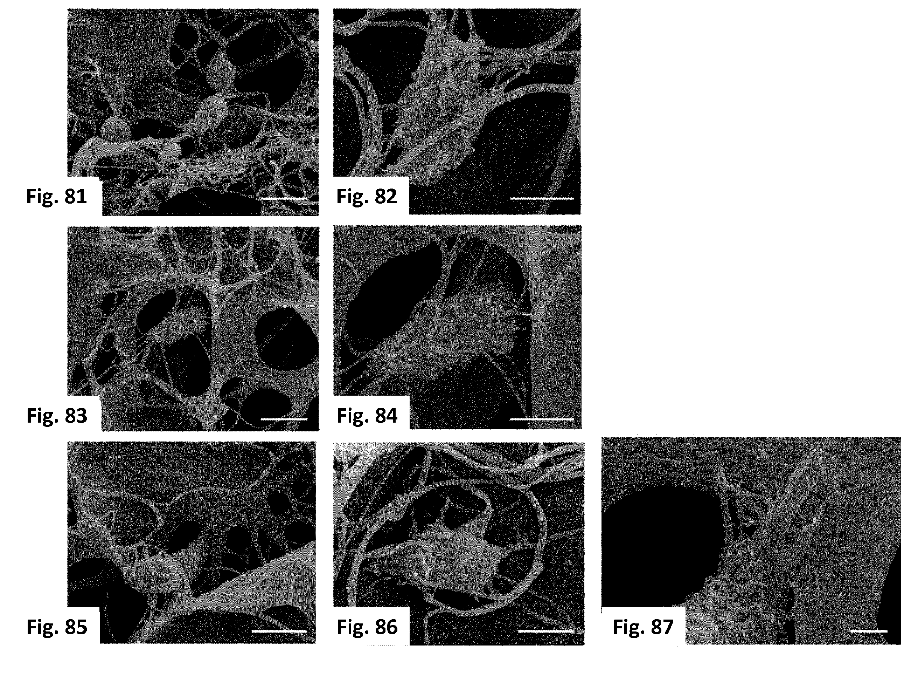

- Figures 81-87: SEM images of NIH 3T3 cells 3 days after seeding on collagen (Figures 81 and 82), on collagen/F50 nanoparticle 10/1 material (Figures 83 and 84) or on collagen/F120 nanoparticle 10/1 material (Figures 85 to 87) (prepared according to example 1.5). The scale bar represents 10 $\mu$m for Figures 81, 83 and 85 (magnification 1,900 for Figure 81, 2,000 for Figure 83, 2,200 for Figure 85); the scale bar represents 5 $\mu$m for Figures 82, 84 and 86 (magnification 5,500 for Figure 82, 5,000 for Figure 84, 4,300 for Figure 86); the scale bar represents 1 $\mu$m for Figure 87 (magnification 13,000).

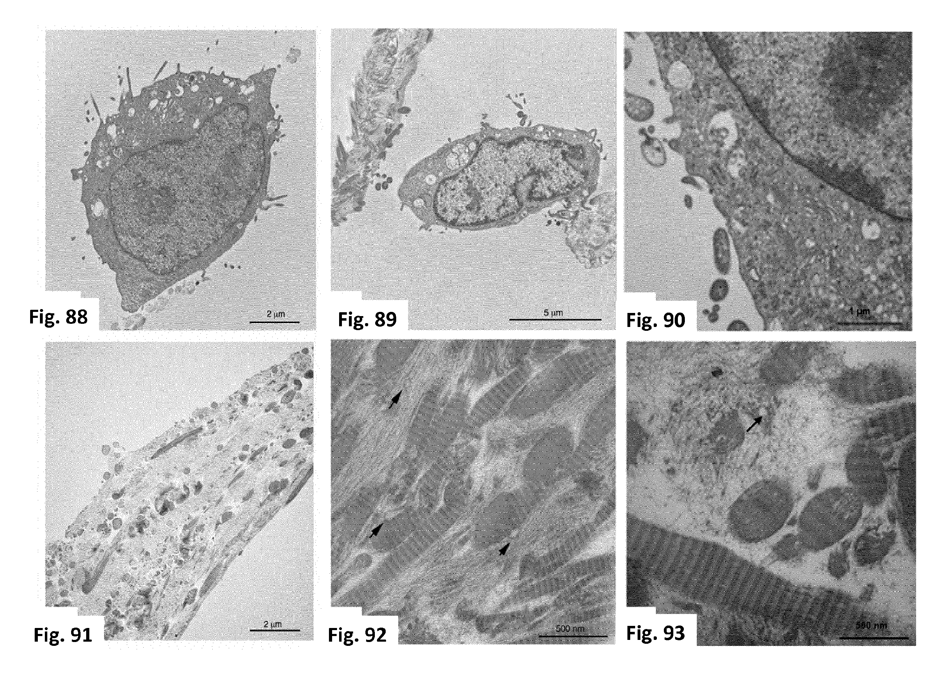

- Figures 88-93: TEM images of NIH 3T3 cells 3 days after seeding on collagen (Figures 88 and 91), on collagen/F50 nanoparticle 10/1 material (Figures 89 and 92) or on collagen/F120 nanoparticle 10/1 material (Figures 90 and 93) (prepared according to example 1.5). Particles are evidenced by arrows.

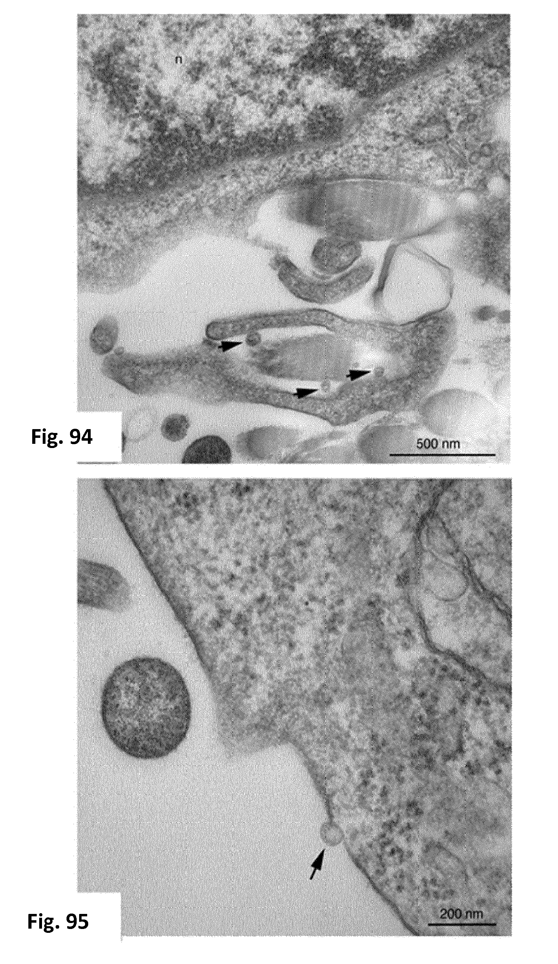

- Figures 94-95: TEM images of points of interaction between a NIH 3T3 cell and collagen/F50 nanoparticle 10/1 material 3 days after seeding. 50 nm particles are evidenced by arrows.

## EXAMPLES

## Example 1. Material preparation

1.1. Preparation of **collagen** gel

Collagen of type I was extracted from equine Achilles tendon.
A collagen gel was then prepared by mixing 1%wt of the obtained collagen with water. Acetic acid was added as a preservative and to obtain a pH of 3.

## 1.2. Preparation of oil-in-water nanoemulsions

1.2.1. Preparation of F50 and F120 nanoemulsions

Two oil-in-water emulsions comprising nanoparticles having different nanoparticle sizes were prepared: F50 (50 nm diameter) and F120 (120 nm diameter) nanoemulsions comprising the compounds listed in the following table 1.

Table 1: Composition of the F50 and F120 emulsions

| Compound | Provider | Quantity (mg) | |
|---|---|---|---|
| | | F50 emulsion | F120 emulsion |
| refined soybean oil | Croda Uniqema, France | 85 | 150 |
| Suppocire NC™ | Gatefosse, France | 255 | 450 |
| Lipoid S75™ | Lipoid, Germany | 65 | 45 |
| Myrjs40™ | Croda Uniqema, France | 345 | 215 |
| aqueous medium: 1X PBS or 154 mM NaCl | | qsp 2 ml | qsp 2 ml |

An oily premix was prepared by mixing refined soybean oil, Suppocire NC™, and Lipoid S75™. After homogeneization at 50°C, the continuous aqueous phase, composed of Myrjs40™ and of aqueous medium, was introduced. The vial was placed in a 50°C water bath and the mixture was sonicated for 5 min using a VCX750 ultrasonic processor (power output 190 W, 3-mm probe diameter, Sonics). The obtained emulsions were dialyzed overnight at room temperature (20°C) against 1000 times their volume in the appropriate aqueous buffer (12-14,000 Da MW cut off membranes, ZelluTrans).
Finally, the emulsions were filtered through a 0.22 $\mu$m Millipore membrane for F50 emulsion and through 0.45 $\mu$m Millipore membrane for F120 emulsion.

### 1.2.2. Preparation of F50 and F120 emulsions the nanoparticles of which comprise DiI/DiD FRET fluorophore pair

A FRET dye pair is constituted of a donor dye (here 1,1'-Dioctadecyl-3,3,3',3'-Tetramethylindocarbocyanine Perchlorate (DiI)) which emission spectrum overlaps with an acceptor dye (here 1,1'-Dioctadecyl-3,3,3',3'-Tetramethylindodicarbocyanine Perchlorate (DiD)) absorption spectrum. When the two fluorophores are in close proximity (ie a few nm), the emission of the donor (DiI) is quenched and transferred to the acceptor (DiD), when excitation is in the donor range. The co-encapsulation of a FRET dye pair in the lipid core of the nanoparticles can therefore be used to monitor the nanoparticle core integrity, measuring the FRET signal, quantified as the acceptor/donor (DiD/DiI) fluorescence ratio.

FRET F50 and F120 emulsions were prepared as described previously, by adding:

- 40 $\mu$L of DiI at 10 mM in EtOH (Life Technologies, France) and 160 $\mu$L of DiD at 10 mM in EtOH (Life Technologies, France) for FRET F120 emulsion, or
- 20 $\mu$L of DiI at 10 mM in EtOH and 80 $\mu$L DiD at 10 mM in EtOH for FRET F50 emulsion

in the oily premix, prepared otherwise exactly as previously described.

The absorbance and fluorescence measurements were performed in 1X PBS, respectively, using a Cary 300 ScanUV-Visible spectrophotometer (Varian) and a Perkin Elmer LS50B fluorimeter.

The DiD/DiI fluorescence ratio of FRET F50 emulsion was 0.709 and the one of FRET F120 emulsion was 2.621. The ratios obtained after having added DMSO to the emulsions were 0.207 for FRET F50 emulsion and 0.16 for FRET F120 emulsion. The difference between the DiD/DiI fluorescence ratio of FRET F50 emulsion and the one of FRET F120 emulsion is due to the lower concentration of DiD in FRET F50 emulsion, which makes the FRET process efficiency far lower for FRET F50 emulsion than for FRET F120 emulsion.

Adding DMSO dissociated the nanoparticles of the emulsions and causes the drop of the DiD/DiI fluorescence ratio. As expected, the DiD/DiI fluorescence ratio is an excellent indicator of the integrity of the lipid core of the nanoparticles.

### 1.3. Characterization of oil-in-water emulsions

The emulsions were characterized by their size and polydispersity index, using Dynamic Light Scattering (Zeta Sizer Nano ZS, Malvern Instrument). For this purpose, the emulsions were diluted to achieve 2 mg/ml of components of the nanoparticles (ie: combination of oil + Suppocire + Lipoid + Myrjs40) in sterile PBS 0.1X and were transferred in Zeta Sizer Nano cells (Malvern Instrument) before each measurement, performed in triplicate.

Results are described in Table 2.

Table 2. Nanoparticle diameter, Pdl and zeta potential of the prepared emulsions

| Emulsion | Nanoparticles concentration (mg/mL) | Nanoparticle diameter (nm) | Polydispersity Index (Pdl) | Zeta potential (mV) |
|---|---|---|---|---|
| F50 | 100 | 47 ± 1 | 0.181 ± 0.001 | |
| F120 | 100 | 116 ± 2 | 0.163 ± 0.006 | |
| FRET-F50 | 100 | 63 ± 1 | 0.174 ± 0.006 | -8.23 ± 0.705 |
| FRET-F120 | 100 | 123 ± 2 | 0.133 ± 0.010 | -6.75 ± 0.086 |

The F50, F120 and FRET emulsions were stable more than 5 months at 4°C (the nanoparticle diameters and Pdl thereof did not change).

### 1.4. Stability of FRET emulsions in the collagen gel

To assess the chemical stability of the nanoparticles of the emulsions after their inclusion in the collagen gel, FRET experiments were carried out. DiI/DiD FRET F50 and F120 emulsions at a concentration of 10%wt of nanoparticles in PBS buffer were loaded in the collagen gel prepared in 1.1, thus obtaining a 0.1:1:98.9 w/w/w nanoparticle:collagen:buffer mixture.

The size, polydispersity and emission spectra of the FRET nanoparticles dispersed in the collagen gel were monitored through time when stored at room temperature (20°C). For this purpose, at desired time points, 400 mg of collagen gel were dispersed in 1 mL deionized water and fluorescence spectra recorded using a Perkin Elmer LS50B fluorimeter. The size, polydispersity index and zeta potential of the solutions were recorded as well using a ZetaSizer (Malvern). Negative controls with FRET F120 and FRET F50 nanoparticles dispersed in 1X PBS in the same diluted conditions as the gels were used (nanoparticle concentration of 0.1 % w:v).

No fluorescence signal was observed for the collagen gel alone, contrarily to nanoparticle samples. Results are summarized in Table 3.

Table 3: nanoparticle diameter, PdI, zeta potential and DiD/DiI fluorescence ratio of F50 and F120 nanoparticles before and after introduction into the collagen gel

| | F50 | | | | F120 | | | |
|---|---|---|---|---|---|---|---|---|
| | Δ | PDI | ζ | ratio | Δ | PDI | ζ | ratio |
| before introduction of the emulsion in the gel (Just after their preparation) | 63 ± 1 | 0.17 ± 0.01 | -8.2 ± 0.7 | 0.71 ± 0.07 | 123 ±2 | 0.13 ± 0.01 | -6.7 ± 0.1 | 2.6 ± 0.3 |
| After 120h in 1X PBS, in the absence of collagen | 66.3± 1.5 | 0.24 ± 0.02 | -8.7 ± 0.1 | 0.79 ± 0.08 | 123 ± 2 | 0.16 ± 0.01 | -11.1 ± 3.5 | 2.2 ± 0.2 |
| After 24h in collagen gel | 129.2 ± 4.7 | 0.39 ± 0.01 | +12.0 ± 1.6 | 1.8 ± 0.2 | 161 ±7 | 0.34 ± 0.03 | +14.9 ± 1.2 | 4.5 ± 0.5 |
| After 48h in collagen gel | 168.4 ± 4.4 | 0.45 ± 0.02 | | 2.6 ± 0.3 | 210 ±5 | 0.4 ± 0.004 | | 2.9 ± 0.3 |
| "Nominal" parameters when embedded in lyophilized matrix after 1 h mixing in the gel form | 168 ± 3 | 0.26 ± 0.01 | | | 172 ± 9 | 0.25 ± 0.03 | | |
| Δ: diameter in nm ζ: zeta potential in mV Ratio: DiD/DiI fluorescence ratio | | | | | | | | |

The nanoparticles were stable for 5 days in diluted 1X PBS conditions (and more than 5 months in more concentrated storage conditions).

When the nanoparticles were incubated in the collagen gel, we observed a coating of the nanoparticles by the collagen fibers that induced an increase in particle size and size dispersity, as well as a shift from negatively-charged nanoparticle surface to positively-charged nanoparticle surface.

After 48 hours in collagen gel, particle size and polydispersity had increased too much to consider the formulation any longer stable for the F120 nanoparticles (DLS results are poor quality). At day 5, no clear size peak could be determined by DLS, either for F50 or F120. Therefore, colloidal stability of the nanoparticles in the collagen gel form was ensured for around 24h.

The DiD/DiI fluorescence ratio reflects the integrity of the nanoparticle core. We would have expected a decrease of the fluorescence ratio with the destabilization of the nanoparticles, as evidenced in example 1.2.2. when DMSO was added. It is what was observed for F120 nanoparticles when highly diluted in the buffer (without collagen) especially after day 9, when the nanoparticles start to dissociate since they are quite highly diluted. F50 nanoparticles dispersed in PBS buffer were more stable. A decrease of the DiD/DiI FRET ratio for the F120 and F50 nanoparticles dispersed in the collagen gel starting at day 1 for F120, day 2 for F50 was also observed, as correlated with destabilization observed by DLS. However, more surprisingly, this loss of the FRET signal was preceded by an increase of the DiD/DiI fluorescence ratio: dispersion of the nanoparticles in the collagen gel made the FRET process more efficient, meaning the DiD and DiI fluorophores were concentrated in a smaller volume in which they interacted better. It could be due to the fact that DiD/DiI fluorophores could migrate more in the core of the nanoparticles when collagen fibers adsorbed on their surface, and therefore interacted stronger, or to a shrinking of the lipid core of the nanoparticles when dispersed in the collagen material.

In conclusion, F50 and F120 nanoparticles were stable in the collagen gel (when loaded at 0.1:1:98.9 nanoparticle:collagen:buffer w/w/w) for 24 hours. This is a far enough sufficient time lapse to ensure the good homogenization of the collagen/nanoparticles samples before their lyophilization to obtain dry materials.

**1.5. Collagen / nanoparticles material preparation according to method A:**

**Dispersion of the emulsion in the collagen gel followed by lyophilization**

Collagen / nanoparticles materials were prepared from the emulsions prepared in examples 1.2.1 or 1.2.2.

The material was prepared loading in the collagen gel prepared in 1.1 two different sizes of nanoparticles: F50 and F120 nanoparticles, with and without DiI/DiD, at the concentration of 10/1 w/w dry collagen/lipids. More precisely, 25 g of collagen gel (containing 250 mg of dry collagen), 2.25 mL of milliQ water and 250 $\mu$L of either F50 emulsion, or F120 emulsion, or FRET F50 emulsion, or FRET F120 emulsion were mixed at 100 rpm for 1 hour at room temperature (20°C).

A directional freeze-drying procedure under thermally constant freezing conditions was then carried out to provide a sponge like material with a uniform porous microstructure. Either 3 g of gel material were distributed into a 35 mm Petri dish (FALCON Easy Grip Tissue Culture Dish, 353001), or 2 g of gel material were distributed into each well of 12 Well Cell Culture Plate. The air or upper surface was covered with a polypropylene membrane before the freezing. The Petri dish and the 12 Well Plate were placed into a glass container and quickly frozen in liquid nitrogen for 5 min. The freeze-drying process was done for 24 hours with a Cryotec V8.11 plate lyophilizer. After freeze-drying process the polypropylene membrane was removed with a tweezer and the Petri dish or micro-well plate were packed and sealed in a bag for the sterilization process.

The sterilization process was done with gamma irradiation, exposed to a nominal dose of 25 kGy.

Materials with higher nanoparticle payloads were also prepared loading in collagen gel 1% w:w in acetate buffer (Euroresearch) the two different sizes of nanoparticles, F50 and F120 nanoparticles at the concentration of 10/1, 5/1, 3.3/1, 2.5/1, 2/1 w/w dry collagen/lipids. More precisely, respectively 25 g of collagen gel, 2.25 mL of milliQ water and 250 $\mu$L of either F50 emulsion or F120 emulsion at 10% w/v lipids (for concentration 10/1), 25 g of collagen gel, 2.0 mL of milliQ water and 500 $\mu$L of either F50 emulsion or F120 emulsion at 10% w/v lipids (for concentration 5/1), 25 g of collagen gel, 1.75 mL of milliQ water and 750 $\mu$L of either F50 emulsion or F120 emulsion at 10% w/v lipids (for concentration 10/1), 25 g of collagen gel, 1.5 mL of milliQ water and 1000 $\mu$L of either F50 emulsion or F120 emulsion at 10% w/v lipids (for concentration 2.5/1), and 25 g of collagen gel, 1.25 mL of milliQ water and 1250 $\mu$L of either F50 emulsion, or F120 emulsion (for concentration 2/1), were mixed at 100 rpm for 1 hour at room temperature (20°C). The gel materials were processed as described above through freeze-drying to obtain dryed collagen/lipid nanoparticle materials containing respectively 10/1, 5/1, 3.3/1, 2.5/1 and 2/1 w/w dry collagen/lipids.

Control collagen/Myrj™ s40 (collagen/PEG) materials were prepared as well, with collagen/Myrj™ s40 ratio 21/1 and 40/1 weigh/weight. These ratios correspond respectively to the collagen/PEG ratio of the collagen/F50 10/1 material and collagen/F120 10/1 material.

**1.6 Structural characterization of the material obtained in 1.5**

1.6.1. Scanning electron microscopy analysis

The morphology and porosity of the material obtained in 1.5 were explored by Scanning Electron Microscopy (SEM). To this end, the materials were cut with a sterile razor blade into a number of small blocks and attached to aluminum stubs with a carbon tape. Blocks were oriented in such a way as to expose either the upper or the lower surfaces, while others were glued on their longitudinal cut profile. They were sputter-coated with gold in a Balzers MED 010 unit and observed in a JEOL JSM 6010LA electron microscope. Images are presented in Figures 1-6 and Figures 7-12. The organization of collagen fibers revealed an overall trabecular pattern characterized by vertically oriented laminas interconnected by thin filaments. This organization is similar for pure collagen or for collagen/particles materials with collagen/lipid ratio 10/1 w/w (Figures 1-6). The structure appeared slightly less regular with increasing content of nanoparticles, for collagen/lipid ratios below 3.3/1 (Figures 7-12).

The pore sizes were analyzed based on SEM images using the ImageJ® software. Results are listed in Table 4:

Table 4: Pore size analysis of the collagen 100%, collagen/F50 and collagen/F120 at different weight/weight percentages. Collagen and collagen/Myrj™ s40 were used as control materials.

| | number of pores | Evaluated Area ($\mu m^2$) | % Area occupied by pores | Feret's diameter ($\mu m$) |
|---|---|---|---|---|
| Collagen | 168 | $4 \cdot 10^4$ | 48.97 | $87 \pm 58$ |
| Coll F50 10/1 | 121 | $4 \cdot 10^4$ | 37.78 | $99 \pm 62$ |
| CollF120 10/1 | 133 | $4 \cdot 10^4$ | 40.43 | $98 \pm 60$ |

(continued)

|  | number of pores | Evaluated Area ($\mu m^2$) | % Area occupied by pores | Feret's diameter ($\mu m$) |
|---|---|---|---|---|
| CollF50 5/1 | 83 | 4 10$^4$ | 56.91 | 84 $\pm$ 58 |
| CollF120 5/1 | 170 | 4 10$^4$ | 49.11 | 98 $\pm$ 59 |
| CollF50 3.3/1 | 140 | 4 10$^4$ | 52.86 | 94 $\pm$ 59 |
| CollF120 3.3/1 | 93 | 4 10$^4$ | 55.11 | 98 $\pm$ 61 |
| CollF50 2.5/1 | 108 | 4 10$^4$ | 59.13 | 90 $\pm$ 57 |
| CollF120 2.5/1 | 94 | 4 10$^4$ | 53.85 | 95 $\pm$ 62 |
| CollF50 2/1 | 56 | 4 10$^4$ | 25.97 | 119 $\pm$ 57 |
| CollF120 2/1 | 75 | 4 10$^4$ | 56.08 | 107 $\pm$ 62 |
| Coll/Myrj 21/1 | 37 | 4 10$^4$ | 43.88 | 96 $\pm$ 61 |
| Coll/Myrj 40/1 | 60 | 4 10$^4$ | 33.71 | 82 $\pm$ 43 |

A fibrous structure with an average pore size of 50-150 $\mu m$ is an ideal structure for collagen material for cell growth. The prepared materials presented this range of porosity, with no significant impact of the coating of the nanoparticles onto the collagen fibers.

1.6.2. Transmission electron microscopy analysis

The ultrastructure of the collagen/nanoparticle materials obtained in example 1.5, and particularly the localization of the nanoparticles inside the matrix, were explored by Transmission Electron Microscopy (TEM). The materials were cut into a number of small blocks with a sterile razor blade and fixed overnight at 4 °C with 3% glutaraldehyde in phosphate buffer (PB) at pH 7.2. Following extensive rinsing with the same buffer at 4 °C, they were immersed for 1h in 0.5 % tannic acid in PB at 4 °C. They were then rinsed again four times in the same buffer for 15 min at 4 °C, and post-fixed for 1h with 1% osmium tetroxide in PB at 4 °C. Specimens were finally washed in distilled water, block-stained with 1% uranyl acetate in distilled water and then dehydrated in a graded series of ethanol.

The dehydrated samples were infiltrated for two days with graded mixtures of LRWhite resin/ethanol. By the end of this procedure, samples were embedded in fresh LRWhite resin and cut with Reichert Ultracut ultramicrotome equipped with a diamond knife. Ultrathin sections (60-80 nm thick) were collected on copper grids, stained with uranyl acetate and lead citrate, and observed with a JEOL 1200 EX II electron microscope. Micrographs were acquired by the Olympus SIS VELETA CCD camera equipped the iTEM software. Images are presented in Figures 13-18, Figures 19-26, Figures 27-34 and Figures 35-38.

For all materials, both the filamentous and the laminar structures of collagen materials appeared comprised of rather compact fibrils characterized by the repeating bandings typical of native collagen, as due to the staggered arrangement of constituting polypeptides, while the nanoparticles appeared regularly distributed with characteristic spherical shape, some of them pointed by arrows in Figures 13 to 34. The nanoparticles had been well highlighted both inside the fibers and along their surface. Evidence of such nanostructures could not be found in materials prepared by mixing the collagen gel with the Myrj™s40 co-surfactant entering the lipid nanoparticle composition then lyophilisation (Figures 35-38), and are specific of the presence of the nanoparticles. Size analysis also confirmed the size of the nanoparticles (50 nm diameter (Figures 19-26) or 120 nm diameter (Figures 27-34)). The images show that the nanoparticle coating did not damage the collagen fiber integrity and organization up to high concentrations of nanoparticles (collagen/lipid ratio 2/1). The ultrastructure of collagen was well maintained, even if the fibers appeared more lax for low collagen/lipid ratios (below 2.5/1).

1.6.3. Confocal laser scanning microscopy analysis

Confocal laser scanning microscopy (CLSM or LSCM) was performed using collagen/ FRET-nanoparticle materials obtained in example 1.5, to confirm the localization of the nanoparticles inside or onto the collagen fibers.

The materials were cut in a small blocks with a sterile bistoury and embedded for 4 hours, at room temperature (20°C), within OCT (compound for Cryostat Sectioning, Tissue-Tek®). The materials were transferred, avoiding the bubbles, in fresh OCT and quickly placed and orientated on cryostat adapter. The samples were directly frozen into the Cryostat (Cryostat Slicing Leica CM 3050S) at -20°C for 3/4 minutes. The sections (10 $\mu$m) were collected on microscope slides Superfrost® plus (Menzel-Glaser, Thermo scientific, J1800AMNZ) and covered with one or two drops of Fluoro-Save™ Reagent (Calbiochem®, Cat. 345789) before placement of coverslip. The images were acquired using three different wavelength excitation, FITC (500-535 nm), TRITC (555-620 nm) and CY5 (650-750 nm). Images are presented in Figures 39-44.

The fluorescence structure, reflecting the nanoparticle distribution, is well homogeneous in both colors (FRET nanoparticles loaded with both DiI and DiD dyes). It reflects the very homogeneous distribution of the nanoparticles through the material. The fluorescent structure also very well match the structure of the collagen fiber network as evidenced by SEM, confirming TEM experiments showing that the nanoparticles are coated on the collagen fibers.

**1.7 Effect of the lyophilization and of the storage on the stability of the nanoparticles of the collagen/nanoparticles materials obtained in 1.5**

1.7.1. Collagenase digestion protocol

The nanoparticles were extracted from the collagen matrix to further characterize their stability during material storage.

To perform analysis on the nanoparticles embedded in the collagen matrix, the following protocol based on collagenase digestion was used. 15 mg of collagenase (Sigma C0130) and 10 mg of calcium chloride (Redi-Dri, Aldrich, ref 746495) were dissolved in 10 mL of PBS (1X, pH 7.4) in a 10 mL glass bottle. 1 mL of this collagenase solution was added to 4 mg of lyophilised material and incubated at 37°C for 3 hours. Non-digested materials, precipitated calcium chloride and collagenase were eliminated by centrifugation at 10 000 g for 2 minutes. The supernatant contained digested collagen chains and nanoparticles. It was analyzed by DLS measurements (particle colloidal stability) and by fluorescence spectroscopy (DiI and DiD localization in the nanoparticles).

1.7.2. Impact of the digestion protocol on the FRET F50 and FRET F120 nanoparticles

FRET-F120 and FRET-F50 nanoparticles were incubated at 37°C for 3 h in the absence or in the presence of collagenase using the same protocol and concentration than used for the lyophilized collagen material. Results are presented in Table 5. Results for the material at day 0 (i.e. just after its preparation) are also included for comparison. Zeta potential could not be measured after collagenase digestion because of the too high ionic strength of the solution; dilution of the media to decrease ionic strength yields to too much diluted solutions to obtain relevant results by DLS. Therefore, only data concerning particle size, size polydispersity, and FRET properties are reported in Table 5.

Table 5: nanoparticle diameter, Pdl and DiD/DiI fluorescence ratio of F50 and F120 nanoparticles in the absence of collagen gel, after incubation with collagenase, just after lyophilisation of the collagen/nanoparticle material obtained in example 1.5 and when 100% dissociation of the nanoparticles by DMSO.

| | F50 | | | F120 | | |
|---|---|---|---|---|---|---|
| | $\Delta$ | PDI | ratio | $\Delta$ | PDI | ratio |
| Emulsion (without collagen) | 63 $\pm$ 1 | 0.17 $\pm$ 0.01 | 0.71 $\pm$ 0.07 | 123 $\pm$ 2 | 0.13 $\pm$ 0.01 | 2.6 $\pm$ 0.3 |
| After 3 hours incubation at 37°C with collagenase (in solution) | 63 $\pm$ 1 | 0.23 $\pm$ 0.01 | 0.46 $\pm$ 0.05 | 125 $\pm$ 2 | 0.21 $\pm$ 0.01 | 1.5 $\pm$ 0.2 |
| Just after lyophilisation of the collagen/nanoparticle material obtained in example 1.5 (day 0) | 129.2 $\pm$ 4.7 | 0.39 $\pm$ 0.01 | 0.43 $\pm$ 0.04 | 161 $\pm$ 7 | 0.34 $\pm$ 0.03 | 1.0 $\pm$ 0.2 |
| When 100% dissociation of the nanoparticles by DMSO (in solution) | NA | NA | 0.21 $\pm$ 0.02 | NA | NA | 0.16 $\pm$ 0.02 |
| $\Delta$: diameter in nm<br>PDI: polydispersity<br>Ratio: DiD/DiI fluorescence ratio | | | | | | |

Comparison of lines 1 and 2 of Table 5 demonstrates that the collagenase treatment necessary for the extraction of the nanoparticles from the material does not impact the colloidal properties of the nanoparticles (except for small increase of particle polydispersity) but impacts their fluorescence properties. The encapsulated dyes are prone to oxidation and degradation, and buffer conditions during incubation can damage their fluorescence emission capabilities. Therefore, to take into account the impact of the extraction protocol when presenting the stability data, in the stability results described hereafter, the FRET data is expressed as % of FRET, calculated as:

$$\% \ \text{FRET} = 100 \ \times \ \frac{\frac{\text{DiD}}{\text{Dil}}\ (\text{t}) - \text{DiD/Dil}\ (100\% \ \text{dissociation})}{\frac{\text{DiD}}{\text{Dil}}\ (\text{t} = 0) - \text{DiD/Dil}\ (100\% \ \text{dissociation})}$$

1.7.3. Stability results

To assess the stability of nanoparticles in the collagen material after lyophilization and upon storage, the FRET nanoparticles were used.

The materials were prepared loading in collagen gel 1% w:w in acetate buffer (Euroresearch) the Dil/DiD loaded FRET F50 and F120 nanoparticles, at the concentration of 10/1 w/w dry collagen/lipids. More precisely, 25 g of collagen gel (containing 250 mg of dry collagen), 2.25 mL of milliQ water and 250 μL of either FRET F50 emulsion, or FRET F120 emulsion were mixed at 100 rpm for 1 hour at room temperature (20°C). Then, 2 g of gel material were distributed into each well of 12 Well Cell Culture Plates. The air or upper surface was covered with a polypropylene membrane before the freezing. The 12 Well Plates were placed into a glass container and quickly frozen in liquid nitrogen for 5 min. The freeze-drying process was done for 24 hours with a Cryotec V8.11 plate lyophilizer. After freeze-drying process the polypropylene membrane was removed with a tweezer and the micro-well plate were packed and sealed in a bag until material analysis.

Table 6 reports the stability results obtained by DLS measurements of the diameter and of the PDI of the FRET nanoparticles extracted from the collagen matrix at different time points of storage. We observed that nanoparticles as analyzed after their embedment in the collagen matrix, then extraction according to the previously described collagenase digestion protocol, both present particle size around 170 nm and polydispersity around 0.25. These values are increased in comparison to reference values obtained in solution. It was previously observed that the hydrodynamic size and polydispersity of the nanoparticles are increased after their embedment in the collagen gel (Table 2) and values obtained after lyophilisation/extraction corresponds to values obtained for the nanoparticles dispersed in the collagen gel. TEM analysis (Figures 13 to 34) evidenced that the nanoparticles retain their nominal size when coating the collagen fibers. It means that it was mainly the extraction and DLS measurement protocols in the presence of collagen fragments and collagenase that were responsible for the observed increase of the values of size and polydispersity. Nevertheless, size and polydispersity values were very stable with time. In conclusion, FRET F50 and F120 nanoparticles embedded in the lyophilized collagen matrices present very good colloidal stability, as assessed by their very stable size and polydispersity, for more than 105 days.

Table 6 also illustrates the stability results obtained for FRET measurements. We observed respectively a ≈ 50% and a = 60 % of FRET signal decrease after material preparation for collagen/F50 10/1 and collagen/F120 10/1 materials then stabilization for 105 days at least.

Table 6: nanoparticle diameter, Pdl and % of FRET of F50 and F120 nanoparticles after their extraction from collagen/lipid nanoparticle 10/1 materials after different time of storage at room temperature.

| | F50 extracted from collagen/F50 10/1 | | | F120 extracted from collagen/F120 10/1 | | |
|---|---|---|---|---|---|---|
| | Δ | PDI | % FRET | Δ | PDI | % FRET |
| Just after lyophilisation | 166.2 ± 2.3 | 0.26 ± 0.01 | 89.6 ± 2.2 | 175.2 ± 1.6 | 0.25 ± 0.01 | 61.8 ± 14.1 |
| After 10 days | 167.4 ± 1.9 | 0.27 ± 0.01 | 50.3 ± 3.2 | 160.9 ± 2.1 | 0.21 ± 0.01 | 37.5 ± 1.8 |
| After 23 days | 169.8 ± 1.6 | 0.26 ± 0.01 | 48.4 ± 2.3 | 182.8 ± 2.5 | 0.28 ± 0.01 | 37.1 ± 1.9 |
| After 50 days | 169.7 ± 2.6 | 0.26 ± 0.01 | - | 168.4 ± 1.5 | 0.24 ± 0.02 | - |
| After 105 days | 171.5 ± 2.6 | 0.25 ± 0.01 | 44.8 ± 5.5 | 172.9 ± 2.5 | 0.24 ± 0.01 | 25.4 ± 4.2 |

(continued)

| | F50 extracted from collagen/F50 10/1 | | | F120 extracted from collagen/F120 10/1 | | |
| --- | --- | --- | --- | --- | --- | --- |
| | Δ | PDI | % FRET | Δ | PDI | % FRET |
| After 192 days | 178.1 ± 1.2 | 0.32 ± 0.01 | 19.4 ± 0.8 | 192.5 ± 1.8 | 0.30 ± 0.02 | 22.5 ± 1.6 |
| Δ: diameter in nm<br>PDI: polydispersity<br>% FRET: as calculated by the equation of paragraph 1.7.2 | | | | | | |

**1.8. Collagen / nanoparticles material preparation according to method B: Synthesis of nanoparticles in collagen gel followed by lyophilization**

In this preparation method, the nanoparticles were formed in situ in the collagen gel, before material lyophilization.

For this purpose, were prepared:

- an oily phase comprising: 68 mg of suppocire, 22.7 mg of soybean oil, 17.3 mg of lecithin + 200 nmol of DiD (for nanoparticle visualization), and
- an aqueous phase comprising: 92 mg of Myrjs40 mixed in 2 mL of distillated water at 50°C, then added to 2 g of collagen gel at 1% w/w in acetic acid at pH 3 (corresponding to 20 mg of dry collagen).

The providers of the different compounds are identical to those detailed in example 1.5. The 2 phases were prepared at 50°C, then mixed together and sonicated in a 50°C water bath for 8 minutes using a VCX750 ultrasonic processor (power output 190 W, 3-mm probe diameter, Sonics).

The nanoparticle dispersion was then dialyzed overnight at room temperature (20°C) against 500 times its volume of distillated water (12-14,000 Da MW cut off membranes, ZelluTrans).

3 g of the sample was then lyophilized after freezing as described in example 1.5.

**1.9 Structural characterization of the material obtained in 1.8**

1.9.1. DLS analysis

The samples obtained in example 1.8. were then analyzed by DLS. Results are reported in Table 7.

Table 7: diameter and PDI of the dispersion of nanoparticles in collagen after dialysis and before lyophilization, and after lyophilization.

| Sample | Hydrodynamic diameter (nm) | Polydispersity index |
| --- | --- | --- |
| Dialyzed sample (before lyophilization) | 61.8 ± 0.4 | 0.20 ± 0.02 |
| Lyophilized sample (after extraction from the material matrix by collagenase digestion according to protocol described in 1.7) (day 0) | 160 ± 4.5 | 0.21 ± 0.02 |

1.9.2. Scanning electron microscopy analysis (SEM) and Transmission electron microscopy analysis (TEM)

SEM images are provided in figures 45 to 47. The obtained material presents a structural organization different than pure collagen material (Figures 1 and 2). However, it displays a high porosity suitable for water draining applications such as dressing use.

**Example 2: Material properties**

**2.1. Swelling ratio and hydrophily/hydrophobicity of materials prepared according to example 1.5 (method A)**

The swelling ratio of the materials, reflecting their water absorbing abilities, was quantified. The materials (collagen as control, collagen +F50 nanoparticles with collagen/lipid ratios 10/1, 3.3/1, and 2/1, collagen + F120 nanoparticles with collagen/lipid ratios 10/1, 3.3/1, and 2/1), prepared according to preparation method A -example 1.5, were cut with a bistoury in small pieces (about 1 cm x 1 cm), weighed and immersed for 24 hours in 5 mL of water or phosphate-buffered saline (PBS) buffer, at room temperature (20°C). Then, the unabsorbable solution was removed by aspiration and the materials were drayed with a filter paper for 3 minutes. Finally, the wet materials were weighed and the swelling ratio (SR) calculated according to the following equation:

$$SR = 100 \times (W_w - W_d) / W_d$$

in which $W_d$ is the weight of dry material (i.e. of the lyophilized material before water or PBS contact) and $W_w$ is the weight of wet drayed material. The results are presented in Table 8 (3 to 6 samples/point):

Table 8: Swelling ratio after 24 hours immersion in either MilliQ water or PBS 1X of collagen as control, collagen +F50 nanoparticles with collagen/lipid ratios 10/1, 3.3/1, and 2/1, collagen + F120 nanoparticles with collagen/lipid ratios 10/1, 3.3/1, and 2/1, prepared according to preparation method A -example 1.5.

| Materials | SR in MilliQ water | SR in 1X PBS |
|---|---|---|
| Collagen | 428 ± 13 | 528 ± 16 |
| Collagen/F50 10/1 | 329 ± 28 | 522 ± 22 |
| Collagen/F50 3.3/1 | 342 ± 15 | 531 ± 64 |
| Collagen/F50 2/1 | 361 ± 31 | 516 ± 44 |
| Collagen/F120 10/1 | 287 ± 12 | 502 ± 25 |
| Collagen/F120 3.3/1 | 334 ± 19 | 520 ± 40 |
| Collagen/F120 2/1 | 333 ± 15 | 507 ± 2 |

Compared to collagen, the swelling ratio of the material according to example 1.5 comprising nanoparticles decreased in water, with a slightly more pronounced effect for F120 than for F50, that could be due to an hydrophobic effect of the lipid nanoparticle coating. No significant effect is observed in 1X PBS, probably because of the presence of salts. However, the materials kept very good absorbing properties, as required for dressing applications.

This more hydrophobic character of collagen/nanoparticles material in comparison to collagen alone observed in MilliQ water was better evidenced at early times of contact between the material and an aqueous phase. When a drop of water (10 μL) was deposited on the material, it was immediately absorbed by the collagen alone, whereas the drops took a tens of seconds to be absorbed for collagen/F50 nanoparticles 10/1 material, even longer for collagen/F120 nanoparticles 10/1 material. This can be quantified using a Digidrop instrument. The water drop contact angle with the material can be measured on photos recorded by a camera at different times after contact; the speed of liquid absorbance inside the materials can also be estimated by analyzing the photos. Results are presented in Table 9.

Table 9: Water drop contact angle and speed of water absorbance of collagen (control), collagen/F50 nanoparticles 10/1, collagen/F120 nanoparticles 10/1, prepared according to preparation method A -example 1.5.

| Sample | Water drop contact angle 10 seconds after drop deposit | Speed of water absorbance |
|---|---|---|
| Collagen (control) | Left angle: 0°<br>Right angle: 0° | < 0.5 s |
| collagen/F50 nanoparticles 10/1 | Left angle: 48.5°<br>Right angle: 47.7° | 21 s |
| collagen/F120 nanoparticles 10/1 | Left angle: 86.0°<br>Right angle: 84.3° | > 90 s |

## 2.2. Release kinetics of the FRET nanoparticles from the collagen

Release of F50-FRET nanoparticles prepared as described in example 1.2.2 from collagen materials was studied for collagen/F50 nanoparticles 20/1 and 10/1. (i.e. 1 and 2 mg of nanoparticules for 20 mg of dry collagen). For this purpose, 2 pieces of each material ($\approx$ 20 mg) were incubated at 37°C in 2 mL of buffer (0.01 M tris-HCl and 50 mM $CaCl_2$). At regular time points, 1 mL of buffer were removed for fluorescence analysis for particle titration (done through a pre-established calibration curve) and replaced by fresh buffer. After 24 h, collagenase (80 $\mu$g/mg of collagen) was added in one solution for each material. Results expressed in % of particles released are summarized in Table 10.

Table 10: % of particles released from the collagen

| Samples non added with collagenase | | | Samples added with collagenase (80 $\mu$g/mg of collagen) after 21 h of incubation | | |
|---|---|---|---|---|---|
| Time (h) | collagen/F50 nanoparticles 20/1 | collagen/F50 nanoparticles 10/1 | Time (h) | collagen/F50 nanoparticles 20/1 | collagen/F50 nanoparticles 10/1 |
| 0 | 0% | 0% | 0 | 0% | 0% |
| - | - | - | 3.5 | 1.7% | 3.3% |
| 21 | 0.7 $\pm$ 1.0% | 3.2 $\pm$ 4.6 % | | | |
| 24 (before collagenase addition) | 0.7 $\pm$ 1.0% | 4.7 $\pm$ 4.3% | 24 | 5.8 % | 10.9 % |
| 25 (after collagenase addition) | 11.9 $\pm$ 3.6 % | 12.8 $\pm$ 2.8 % | - | - | - |
| 26 | 36.1 $\pm$ 7.1 % | 30.5 $\pm$ 0.6 % | - | - | - |
| 27 | 68.8 $\pm$ 9.4 % | 45.7 $\pm$ 3.5 % | - | - | - |
| 46 | 112.0 $\pm$ 20.2 % | 84.3 $\pm$ 12.8 % | 53 | 13.4 % | 18.6% |
| - | - | - | 192 (8 days) | 25.6% | 33.2% |

We observed that the diffusion of the nanoparticles outside of the collagen matrices when swollen in aqueous buffer was slow (about 30% of particles released in 8 days). However, particles were completely released in a few hours when the matrix was enzymatically digested.

### 2.3. Thermal stability

Thermal stability of materials was investigated by Differential Scanning Calorimetry (DSC) using a previously described protocol (P. Angele, J. Abke, R. Kujat, H. Faltermeier, D. Schumann, M. Nerlich, B. Kinner, C. Englert, Z. Ruszczak, R. Mehrl, R. Mueller. "Influence of different collagen species on physico-chemical properties of crosslinked collagen matrices". Biomaterials, vol.25, pp.2831-2841, 2004). 33-45 mg of dry material was immersed for 1h in deionized water, then drayed on filter paper for 1 minute before being sealed in aluminium pans and analyzed using a micro DSC III SETARAM. An aluminium pan with the same weight of deionized water than the wet sample was used as reference. The heating rate was constant at 3°C/min and the temperature was increased from 20 °C to 110°C. Data are summarized in Table 11. Shrinkage temperature was determined as the onset value of the endothermic peak. The thermal denaturation temperature (for helix-to-coil transition of the collagen fibers) was taken at the endothermic peak maximum. The value of denaturation enthalpy was calculated with respect to the weight of collagen in the dried material. Several samples of each material were analyzed.

Table 11: DSC measurements of collagen (control), collagen/F50 nanoparticles 10/1, 3.3/1 and 2/1, collagen/F120 nanoparticles 10/1, prepared according to preparation method A -example 1.5.

| Sample | Shrinkage temperature (onset) (°C) | Thermal denaturation temperature (peak) (°C) | Denaturation enthalpy (J/g) |
|---|---|---|---|
| Collagen | 49.8 | 59.3 | 42.9 |
| Collagen/F50 10/1 | 53.6 | 60.1 | 53.2 |

(continued)

| Sample | Shrinkage temperature (onset) (°C) | Thermal denaturation temperature (peak) (°C) | Denaturation enthalpy (J/g) |
|---|---|---|---|
| Collagen/F50 2/1 | 53.3 | 58.5 | 39.3 |
| Collagen/F120 10/1 | 55.3 | 59.3 | 58.4 |
| Collagen/F120 2/1 | 53.2 | 58.4 | 33.8 |

Particle coating had no impact on thermal denaturation temperature for collagen/F50 10/1 and for collagen/F120 10/1 materials, and a very small one (less than 1°C shift) for high lipid nanoparticle concentrations (Collagen/F50 2/1 and collagen/F120 2/1 materials), indicating the very good preservation of the collagen structure. However, the shrinkage temperature of the material was significantly augmented in the presence of the particle coating (+3°C), demonstrating its protective role for the materials. Similarly, higher denaturation enthalpies were observed for the collagen/F50 10/1 and collagen/F120 10/1 materials, indicating an increased preservation of the helix structure of the collagen fibers of the collagen when coated by the nanoparticles.

## 2.4. Mechanical properties

Uniaxial compression tests were performed on lyophilized cylinder shaped materials (collagen, collagen/F50 10/1, collagen/F120 10/1) of 4.1 mm diameter and 5 mm height using an AR2000EX rheometer (TA instrument) with an initial preload of 0.03 N and a compression speed of 10 $\mu$m/s. The stress, $\sigma$, was calculated as the normal force divided by the surface area of the material, whereas the strain, $\varepsilon$, was the % of compression of the material. The stress-strain plots obtained were typical of an elastic foam-like material (Gibson LJ. Biomechanics of cellular solids. Journal of Biomechanics. 2005;38(3):377-99), and the Young modulus E* of the materials could be calculated as the slopes of stress-strain curves for low strains (linear domain). Values are presented in Table 12.

Table 12: Young modulus of collagen (control), collagen/F50 nanoparticles 10/1, and collagen/F120 nanoparticles 10/1, prepared according to preparation method A -example 1.5., measured during uniaxial compression tests.

| E [kPa] | Sample 1 | Sample 2 | Mean $\pm$ SD |
|---|---|---|---|
| Collagen | 115.676 | 106.958 | 111.4 $\pm$ 6.2 |
| Collagen/F50 10/1 | 95.202 | 51.359 | 73.3 $\pm$ 31.0 |
| Collagen/F120 | 54.985 | 59.205 | 57.1 $\pm$ 3.0 |

For an applied force of defined value (defined stress), the deformation (strain) was more important for collagen/lipid nanoparticle materials than for collagen alone, with a more pronounced effect for collagen/F120 than for collagen/F50. Therefore collagen/lipid nanoparticle materials presented a more elastic behavior than collagen alone.

## 2.5. Biocompatibility

In accordance with the International Organization for Standardization 10993, NIH-3T3 murine fibroblast cells were chosen to perform classical WST-1 cytotoxic assay.

To collect enough cells for plating on collagen-nanoparticles material, NIH 3T3 fibroblasts were first cultured in 75 cm$^2$ flasks containing DMEM (Dulbecco's Modified Eagle's Medium) culture medium, supplemented with 10% of newborn calf serum (NCS) and fetal bovine serum (FBS) respectively, 1% glutamine and 1% penicillin/streptomycin, and maintained in an incubator at 37 °C with 5% $CO_2$ and controlled humidity (Cell incubation time 24h). Upon reaching confluence, cells were passed into new culture vessels in a 1:5 ratio and the medium changed every three days.

Aliquots of 2x10$^5$ cells were seeded on the top of lower surface of the materials (collagen (control), collagen/F50 nanoparticles 10/1, collagen/F120 nanoparticles 10/1, prepared according to preparation method A -example 1.5), on 12 well plate, and incubated at 37 °C in 2 mL of culture medium under a continuous flow of 5% $CO_2$ for 24 h. The WST-1 test measures the cell density through the metabolic activity of mitochondrial enzymes using a substrate that is cleaved to form a colorimetric product which absorbance at 450 nm (A450) is monitored. Cell viability is calculated using formula: % viability= (A450(sample) - A450(T- cytotoxic))/ (A450(positive control) - A450(T-cytotoxic)) x100, where positive control is cell alone, and cytotoxic sample (T- cytotoxic, negative control) is cells incubated in presence of a cytotoxic material (1 cm x 1 cm zinc metal plate)

Results are presented on Table 13. Absorbance reflects cell viability.

Table 13: WST-1 Assay of collagen (control), collagen/F50 nanoparticles 10/1, collagen/F120 nanoparticles 10/1, prepared according to preparation method A -example 1.5.

| Material | % viability |
|---|---|
| Collagen | 99.8 ± 5.5 |
| Collagen/F50 10/1 | 118.6 ± 5.5 |
| Collagen/F120 | 97.4 ± 5.5 |
| Cell incubation time 24h<br>WST-1 Reagent incubation 3h at 37°C<br>Absorbance wavelength 450 nm | |

Cell viability was good in the presence of the different materials. Therefore the collagen/lipid nanoparticle materials are not cytotoxic, either when loaded with F50 nanoparticles or with F120 nanoparticles.

## Example 3: Material use

### 3.1. Cell culture

Human keratinocytes (HaCat) and stabilized murine fibroblasts (NIH 3T3) were cultured in 75 cm$^2$ flasks containing DMEM (Dulbecco's Modified Eagle's Medium) culture medium, supplemented with 10% of newborn calf serum (NCS) and fetal bovine serum (FBS) respectively, 1% glutamine and 1% penicillin/streptomycin, and maintained in an incubator at 37 °C with 5% $CO_2$ and controlled humidity (Cell incubation time 24h). Upon reaching confluence, cells were passed into new culture vessels in a 1:5 ratio and the medium changed every three days.

### 3.2. SEM and TEM protocols

3.2.1 SEM sample preparation and protocol

Collagen/lipid particle scaffolds inclusive of their cellular loads were cut into a number of small blocks with a sterile razor blade and fixed overnight at 4 °C with 3% glutaraldehyde in phosphate buffer (PB) at pH 7.2. Following extensive rinsing with the same buffer at 4 °C, they were immersed for 1h in 0,5% tannic acid in PB at 4 °C. They were then rinsed again four times in the same buffer for 15 min at 4 °C, and post-fixed for 1h with 1% osmium tetroxide in PB at 4 °C. Specimens were finally washed in distilled water, block-stained with 1% uranyl acetate in distilled water and then dehydrated in a graded series of ethanols. The samples were first dried by the critical point method in a Balzers Union CPD 020, sputter-coated with gold in a Balzers MED 010 unit and then observed in a JEOL JSM 6010LA electron microscope.

3.2.2 TEM sample preparation and protocol

Collagen/lipid particle scaffolds inclusive of their cellular loads were prepared as described in section 1.6.1.2.

### 3.3. Cell colonization/3D cell culture matrices

3.3.1 Colonization by HaCat

HaCat cells were seeded on the top of the lower surface of materials (collagen, collagen/F50 nanoparticles 10/1 material, collagen/F120 nanoparticles 10/1 material, prepared according to preparation method A according to example 1.5) prepared in 35 mm Petri dish, with 2x10$^5$ cells/matrix. Materials and cells were then incubated at 37°C in 2 mL of culture medium under a continuous flow of 5% $CO_2$. Medium was then renewed every 3 days.
SEM images 3 days and 7 days after cell seeding are presented in Figures 48-59 and 60-63 respectively. The cells proliferated well on the different matrices. Moreover, whereas the 100% collagen matrix was quite degraded in the presence of the cells, collagen fibers and collagen network was yet clearly identified for the collagen/F50 nanoparticles and the collagen/F120 nanoparticles materials after 3 days (Figures 48-59), and even after 7 days (Figures 60-63). Therefore the collagen/nanoparticles materials are completely suited for 3D cell cultures with moreover a better preservation of the 3D collagen scaffold for longer times.

3.3.2 Colonization by fibroblasts

NIH 3T3 cells were seeded on the top of the lower surface of materials (collagen, collagen/F50 nanoparticles 10/1 material, collagen/F120 nanoparticles 10/1 material, prepared according to preparation method A according to example 1.5) prepared in 35 mm Petri dish, with $2x10^5$ cells/matrix. Materials and cells were then incubated at 37°C in 2 mL of culture medium under a continuous flow of 5% $CO_2$. Medium was then renewed every 3 days.

SEM and TEM images after 4 hours and 3 days of incubation are presented in Figures 64 to 95. All demonstrate that the cells well proliferated on the different matrices.

Already four hours after seeding, the morphology of the cells (SEM images) evidenced a good level of interaction and adhesion, both for the collagen and the collagen/lipid particle materials (Figures 64 to 70). In particular, cytoplasmic protrusions were evidenced between NIH 3T3 cells and collagen/F50 10/1 material (Figure 68). A good level of cell adhesion was also evidenced by TEM (Figures 71 to 74). Figure 71 (enlarged in Figure 73) shows a NIH 3T3 adhesion phase on a collagen/F50 10/1 fiber. Contact points between the cell and the material were better evidenced in Figures 72 and 74. Apparently intact 50 nm diameter particles (arrows) were evidenced into the collagen fibers (Figure 73) and into the fibrillary part of the material (Figure 72 and 74). Figure 75 (enlarged in Figure 78) shows a NIH 3T3 adhesion phase on a collagen/F120 10/1 fiber, with a focus on contact points between the cell and the material in Figures 76 to 80. As for the collagen/F50 material, nanoparticles can be evidenced in the fibrillar portion of the collagen material (Figures 78, 76 and 79) and on the collagen fiber (Figures 77 and 80). Figure 77 (enlarged in Figure 80) shows a cell plasma membrane pseudopodia during the endocytosis phase of a nanoparticle (square). The magnification in Figure 80 better evidences the phagocytosis process by the cell of the 120 nm nanoparticle on the collagen fiber surface.

Three days after seeding, the interactions between cells and materials had increased (Figures 81 to 87). For collagen material, after three days of culture, the enzymatic activity involved in the processing of the material conferred to the collagen fibers a less compact aspect (Figure 91). In contrast, the collagen fibers remained quite dense for the collagen/F50 10/1 material (Figure 92) and for the collagen/F120 10/1 material (Figure 93). Nanoparticles, either 50 nm diameter (Figure 92) or 120 nm diameter (Figure 93) were still clearly visible among the collagen fibers (indicated by arrows). Interestingly, cells seemed to incorporate portions of collagen fibers as demonstrated in Figures 94 and 95 focussing on a point of interaction between a NIH 3T3 cell and collagen/F50 10/1 material. Figures 94 and 95 show cell cytoplasmic protrusions enveloping collagen fibers and F50 nanoparticles (arrows); Figure 95 shows a F50 nanoparticle in contact with the cell membrane (arrow).

In conclusion, for both keratinocytes and fibroblasts, all the matrices (collagen, collagen/F50 nanoparticle 10/1, collagen/F120 nanoparticle 10/1) displayed good adhesion properties with the cells. In addition, the collagen fibers seemed to be more resistant to the enzymatic degradation involved in the processing of the material in the collagen/lipid nanoparticle materials (Figures 48-59, 60-63, 88-93), and the cells seemed to be able to internalize for further processing both the collagen material and the nanoparticles (Figures 94-95). Therefore in the case the nanoparticle vehicle a payload, this latter should be delivered into the cells.

## Claims

1.  Process for the preparation of a material comprising collagen the fibers of which are coated with nanoparticles, comprising the steps consisting in:

     1) providing an aqueous dispersion comprising collagen and nanoparticles comprising:

     - an amphiphilic lipid,
     - a solubilising lipid comprising at least one fatty acid glyceride, and
     - a co-surfactant comprising at least one poly(ethylene oxide) chain,

     2) drying the aqueous dispersion of step 1) to obtain the material comprising collagen the fibers of which are coated with nanoparticles.

2.  Process according to claim 1, wherein the aqueous dispersion provided in step 1) is obtained by a process comprising the steps consisting in:

     A1) providing a nanoemulsion comprising a continuous aqueous phase and nanoparticles comprising the amphiphilic lipid, the solubilising lipid and the co-surfactant,

A2) mixing the nanoemulsion obtained in step 1a) with collagen to obtain the aqueous dispersion comprising collagen and nanoparticles.

3. Process according to claim 2, wherein the nanoemulsion of step A1) is obtained by a process comprising:

A1a) preparing an oily phase comprising the solubilising lipid and the amphiphilic lipid,
A1b) preparing an aqueous phase comprising the co-surfactant,
A1c) dispersing the oily phase in the aqueous phase under the action of sufficient shear to form a nanoemulsion.

4. Process according to claim 1, wherein the aqueous dispersion provided in step 1) is obtained by a process comprising the steps consisting in:

B1) preparing an oily phase comprising the solubilising lipid and the amphiphilic lipid,
B2) preparing an aqueous phase comprising the co-surfactant and collagen,
B3) dispersing the oily phase in the aqueous phase under the action of sufficient shear to obtain the aqueous dispersion comprising collagen and nanoparticles.

5. Process according to anyone of claims 1 to 4, wherein drying in step 2) is carried out by freeze-drying.

6. Process according to anyone of claims 1 to 5, comprising, between steps 1) and 2), a step 1bis) of applying the dispersion of step 1) to a substrate.

7. Process according to anyone of claims 1 to 6, wherein the amphiphilic lipid is a phospholipid.

8. Process according to anyone of claims 1 to 7, wherein the solubilising lipid is a mixture of glycerides of saturated fatty acids comprising at least 10 % by weight of C12 fatty acids, at least 5 % by weight of C14 fatty acids, at least 5 % by weight of C16 fatty acids and at least 5 % by weight of C18 fatty acids.

9. Process according to anyone of claims 1 to 8, wherein the nanoparticles further comprise an oil.

10. Material comprising collagen the fibers of which are coated with nanoparticles obtainable by the process according to anyone of claims 1 to 9.

11. Material comprising collagen the fibers of which are coated with nanoparticles comprising a solubilising lipid comprising at least one fatty acid glyceride, an amphiphilic lipid, and a co-surfactant comprising at least one poly(ethylene oxide) chain, wherein the material comprises pores the Feret's diameter of which are between 50 and 150 $\mu$m.

12. Material according to claim 10 or 11, wherein the weight of collagen compared to the weight of the nanoparticles is from 0.05/1 to 1000/1.

13. Material according to anyone of claims 10 to 12, comprising less than 25%wt of water.

14. Material according to anyone of claims 10 to 13, the swelling ratio of which in 1X PBS is from 70 to 100%, preferably from 80 to 100%, more preferably from 90 to 100% of the swelling ratio of collagen.

15. Use of the material according to anyone of claims 10 to 14 for wound healing dressings, 3D cell culture materials, tissue engineering, as a sensor, in diagnostic, pharmaceutical, or cosmetic industry.

Fig. 1

Fig. 2

Fig. 3

Fig. 4

Fig. 5

Fig. 6

Fig. 8

Fig. 10

Fig. 12

Fig. 7

Fig. 9

Fig. 11

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

Fig. 32

Fig. 33

Fig. 34

Fig. 36

Fig. 38

Fig. 35

Fig. 37

**Fig. 39**

**Fig. 40**

**Fig. 41**

**Fig. 42**

**Fig. 43**

**Fig. 44**

Fig. 47

Fig. 46

Fig. 45

Fig. 48

Fig. 49

Fig. 50

Fig. 51

Fig. 52

Fig. 53

Fig. 54

Fig. 55

Fig. 56

Fig. 57

Fig. 58

Fig. 59

Fig. 60

Fig. 61

Fig. 62

Fig. 63

Fig. 64

Fig. 65

Fig. 66

Fig. 67

Fig. 68

Fig. 69

Fig. 70

**Fig. 71**

**Fig. 72**

**Fig. 73**

**Fig. 74**

Fig. 77

Fig. 80

Fig. 76

Fig. 79

Fig. 75

Fig. 78

Fig. 87

Fig. 82

Fig. 84

Fig. 86

Fig. 81

Fig. 83

Fig. 85

Fig. 90

Fig. 93

Fig. 89

Fig. 92

Fig. 88

Fig. 91

**Fig. 94**

**Fig. 95**

Europäisches Patentamt
European Patent Office
Office européen des brevets

# EUROPEAN SEARCH REPORT

Application Number

EP 15 30 5894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 199 03 717 A1 (HENKEL KGAA [DE]) 3 August 2000 (2000-08-03) * page 2, lines 37-40 * * page 3, lines 13-39 * * page 3, lines 47-51 * * page 3, lines 56-57 * * page 3, lines 61-63 * ----- | 10-15 | INV. A61L15/32 A61L15/34 C12N5/00 A61L15/52 A61L15/42 |
| X | GUILIN WANG ET AL: "Bisphosphonate-decorated lipid nanoparticles designed as drug carriers for bone diseases", JOURNAL OF BIOMEDICAL MATERIALS RESEARCH PART A, vol. 100A, no. 3, 30 March 2012 (2012-03-30), pages 684-693, XP055126576, ISSN: 1549-3296, DOI: 10.1002/jbm.a.34002 * page 685, right-hand column, line 25 - page 686, right-hand column, line 6 * * page 686, left-hand column, line 49 - page 687, left-hand column, line 7 * * abstract * ----- | 10-15 | |
| A | GRÁINNE M CUNNIFFE ET AL: "Development and characterisation of a collagen nano-hydroxyapatite composite scaffold for bone tissue engineering", JOURNAL OF MATERIALS SCIENCE: MATERIALS IN MEDICINE, KLUWER ACADEMIC PUBLISHERS, BO, vol. 21, no. 8, 20 December 2009 (2009-12-20), pages 2293-2298, XP019822023, ISSN: 1573-4838 * page 2297, left-hand column, lines 8-16 * * abstract; figure 3 * ----- | 1-15 | TECHNICAL FIELDS SEARCHED (IPC)<br><br>A61L C12N |

The present search report has been drawn up for all claims

| Place of search | Date of completion of the search | Examiner |
|---|---|---|
| The Hague | 17 November 2015 | Zalfen, Alina |

EPO FORM 1503 03.82 (P04C01)

## ANNEX TO THE EUROPEAN SEARCH REPORT
## ON EUROPEAN PATENT APPLICATION NO.

EP 15 30 5894

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2015

| Patent document cited in search report | Publication date | Patent family member(s) | Publication date |
|---|---|---|---|
| DE 19903717 A1 | 03-08-2000 | AT 296086 T<br>AU 4521800 A<br>DE 19903717 A1<br>EP 1143926 A1<br>WO 0044343 A1 | 15-06-2005<br>18-08-2000<br>03-08-2000<br>17-10-2001<br>03-08-2000 |

EPO FORM P0459

For more details about this annex : see Official Journal of the European Patent Office, No. 12/82

## REFERENCES CITED IN THE DESCRIPTION

**Patent documents cited in the description**

- WO 2010018223 A
- WO 2011101602 A
- WO 2013144369 A

**Non-patent literature cited in the description**

- European Pharmacopoeia. 2002
- **MARTINDALE.** The Extra Pharmacopoeia. 1996
- **C. J. DOILLON ; C. F. WHYNE ; S. NBRANDWEIN ; F. H. SILVER.** Collagen-based wound dressings: control of the pore structure and morphology. *J. Biomed. Mater. Res.,* 1986, vol. 20, 1219-1228
- **P. ANGELE ; J. ABKE ; R. KUJAT ; H. FALTERMEIER ; D. SCHUMANN ; M. NERLICH ; B. KINNER ; C. ENGLERT ; Z. RUSZCZAK ; R. MEHRL.** Influence of different collagen species on physico-chemical properties of crosslinked collagen matrices. *Biomaterials,* 2004, vol. 25, 2831-2841
- **GIBSON LJ.** Biomechanics of cellular solids. *Journal of Biomechanics,* 2005, vol. 38 (3), 377-99